(19) Europäisches Patentamt
European Patent Office
Office européen des brevets

(11) Veröffentlichungsnummer: **0 277 611 B1**

(12) **EUROPÄISCHE PATENTSCHRIFT**

(45) Veröffentlichungstag der Patentschrift: **16.06.93**

(51) Int. Cl.$^5$: **C07D 405/04**, C07D 413/04, C07D 417/04, A61K 31/40, A61K 31/41, A61K 31/535, A61K 31/54

(21) Anmeldenummer: **88101344.5**

(22) Anmeldetag: **30.01.88**

# (54) Alkylsubstituierte N-Benzopyranyllactame, Verfahren zu ihrer Herstellung, ihre Verwendung sowie pharmazeutische Präparate auf Basis dieser Verbindungen.

(30) Priorität: **04.02.87 DE 3703229**
**28.07.87 DE 3724876**

(43) Veröffentlichungstag der Anmeldung:
**10.08.88 Patentblatt 88/32**

(45) Bekanntmachung des Hinweises auf die Patenterteilung:
**16.06.93 Patentblatt 93/24**

(84) Benannte Vertragsstaaten:
**AT BE CH DE ES FR GB GR IT LI LU NL SE**

(56) Entgegenhaltungen:
**EP-A- 0 076 075**
**EP-A- 0 107 423**
**EP-A- 0 120 427**
**EP-A- 0 120 428**
**EP-A- 0 173 848**
**EP-A- 0 273 262**
**EP-A- 0 277 612**

**JOURNAL OF MEDICINAL CHEMISTRY, Band 29, 1986, American Chemical Society, Washington, DC, US; V.A. ASHWOOD et al.: "Synthesis and antihypertensive activity of 4-(cyclic amido)-2H-1-benzopyrans", Seiten 2194-2201**

(73) Patentinhaber: **HOECHST AKTIENGESELLSCHAFT**
**Postfach 80 03 20**
**W-6230 Frankfurt am Main 80(DE)**

(72) Erfinder: **Englert, Heinrich Christian, Dr.**
**Stormstrasse 13**
**W-6238 Hofheim am Taunus(DE)**
Erfinder: **Lang, Hans-Jochen, Dr.**
**Rüdesheimer Strasse 7**
**W-6238 Hofheim am Taunus(DE)**
Erfinder: **Mania, Dieter, Dr.**
**Berliner Ring 5**
**W-6233 Kelkheim (Taunus)(DE)**
Erfinder: **Schölkens, Bernward, Dr.**
**Hölderlinstrasse 62**
**W-6233 Kelkheim (Taunus)(DE)**
Erfinder: **Klaus, Erik, Dr.**
**Parkstrasse 12**
**W-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 277 611 B1

Rank Xerox (UK) Business Services
(3.10/3.6/3.3.1)

## Beschreibung

Die Erfindung betrifft N-Benzopyranyllactame der Formel I

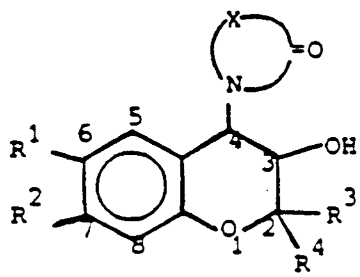

I,

in welcher

- $R^1$ für CN, $NO_2$, $SO_n$-($C_1$-$C_6$)-Alkyl, $SO_n$-Ar, steht; wobei n = 1 oder 2, Ar für Phenyl steht, das unsubstituiert oder durch 1 bis 3 gleiche oder verschiedene Reste ($C_1$-$C_2$)-Alkyl, ($C_1$-$C_2$)Alkoxy, Halogen, Trifluormethyl, CN, $NO_2$, CO-($C_1$-$C_2$)-Alkyl, $SO_p$-($C_1$-$C_2$)Alkyl, substituiert ist und p für 1 oder 2 steht,
- $R^2$ für H, OH, ($C_1$-$C_2$)-Alkoxy, ($C_1$-$C_2$)-Alkyl, Halogen oder $NR^5R^6$ steht, wobei $R^5$ und $R^6$ gleich oder verschieden sind und für H, ($C_1$-$C_2$)Alkyl oder ($C_1$-$C_2$)-Alkylcarbonyl stehen,
- $R^3$ und $R^4$ gleich oder verschieden sind und für Alkyl mit 1-4 C-Atomen stehen,

die oben genannten Bedeutungen von $R^1$ und $R^2$ auch vertauscht sein können,

- X für eine Kette $(CH_2)_m$ steht, die durch mindestens 1 und höchstens 2m-1 ($C_1$-$C_2$)-Alkylgruppen substituiert ist, sowie durch ein Heteroatom Y in der Bedeutung von O, $NR^7$, S unterbrochen sein kann und $R^7$ H oder ($C_1$-$C_4$)-Alkyl bedeutet und m für 2, 3 oder 4 steht, wobei die Konfiguration an $C_3$ und $C_4$ stets entgegengesetzt ist.

Unter Halogen wird F, Cl, Br oder J, vorzugsweise F und Cl verstanden.

Die C-Atome 3 und 4 des 3,4-Dihydro-2H-benzo[b]pyransystems (nachfolgend auch als "Chromansystem" bezeichnet) der Formel I sind asymmetrisch substituiert. Die Erfindung betrifft nur solche Verbindungen, die an diesen Zentren entgegengesetzte Konfigurationen aufweisen. Dies bedeutet, daß der Lactamring als Substituent an C-4 und die OH-Gruppe an C-3 stets "trans" zueinander orientiert sind. Die oben genannte Definition von X bedeutet, daß außerdem der Lactamring mindestens ein, höchstens jedoch m (mit m in der eingangs erwähnten Definition) chirale C-Atome enthält. Die Erfindung betrifft dabei sowohl Verbindungen mit R-als auch S-konfigurierten Zentren. Gleiches gilt für den Fall, daß $R^1$, $R^2$, $R^3$ oder $R^4$ Asymmetriezentren enthalten oder selbst als Substituenten ein Asymmetriezentrum erzeugen. Die Verbindungen können dann als optische Isomere, als Diastereoisomere, als Racemate oder als Gemisch derselben vorliegen.

Bevorzugt sind Verbindungen der Formel I, in denen
$R^1$, $R^2$, $R^3$, $R^4$ die oben genannten Bedeutungen aufweisen und X für eine Kette $(CH_2)_m$ steht; die durch eine ($C_1$-$C_2$)-Alkylgruppe substituiert ist, sowie durch ein Heteroatom Y, das für O, S oder $NR^7$ steht mit $R^7$ in der Bedeutung von H oder ($C_2$-$C_4$)-Alkyl, unterbrochen sein kann und wobei m für 2, 3 oder 4 steht.

Weiter bevorzugt sind Verbindungen der Formel I, in welcher
$R^1$ bis $R^4$ die oben genannten Bedeutungen aufweisen und X für eine Kette $(CH_2)_m$ steht, die durch eine ($C_1$-$C_2$)-Alkylgruppe substituiert ist wobei m für 3 oder 4 steht.

Ganz besonders bevorzugt sind dabei solche Verbindungen I, bei denen $R^1$ bis $R^4$ die oben genannten Bedeutungen aufweisen und X für eine Kette $(CH_2)_m$ steht, wobei m für 3 oder 4 steht, die durch eine ($C_1$-$C_2$)-Alkylgruppe an dem C-Atom substituiert ist, das dem N-Atom des Lactamringes benachbart ist.

Insbesondere bevorzugt sind Verbindungen, bei denen $R^1$-$R^4$ die oben genannten Bedeutungen aufweisen und X für eine Kette $(CH_2)_m$ steht mit m = 3 oder 4, die durch eine ($C_1$-$C_2$)-Alkylgruppe an dem C-Atom substituiert ist, das dem N-Atom des Lactamringes benachbart ist, und zwar derart, daß die Konfiguration dieses C-Atoms dieselbe ist wie das des C-4 Atom des Chromansystems.

Ebenfalls ganz besonders bevorzugt sind Verbindungen I, bei denen
$R^1$ für CN oder $SO_2$-$CH_3$ und $R^2$ für H steht,
$R^3$ und $R^4$ gleich oder verschieden sind und für Alkyl mit 1 bis 2 C-Atomen stehen,
X für eine Kette $(CH_2)_m$ steht mit m = 3 oder 4, die durch eine ($C_1$-$C_2$)-Alkylgruppe an dem C-Atom

substituiert ist, das dem N-Atom des Lactamringes benachbart ist, und zwar derart, daß die Konfiguration dieses C-Atoms dieselbe ist wie das des C-4 Atoms im Chromansystem;
ebenso bevorzugt sind Verbindungen I, bei denen $R^1$ für $SO_2$- Phenyl, das unsubstituiert oder durch 1 bis 3 Substituenten wie oben erwähnt substituiert ist,
$R^2$ für H oder $OCH_3$ steht,
$R^3$ und $R^4$ gleich sind oder verschieden und für $(C_1-C_2)$-Alkyl stehen,
X für eine Kette $(CH_2)_m$ steht mit m = 3 oder 4, die durch eine $(C_1-C_2)$-Alkylgruppe an dem C-Atom substituiert ist, das dem N-Atom des Lactamringes benachbart ist und zwar derart, daß die Konfiguration dieses C-Atoms dieselbe ist wie die des C-4-Atoms im Chromansystems.

Bevorzugt sind auch Verbindungen, bei denen $R^1$-$R^4$ die oben genannten Bedeutungen aufweisen und X für eine Kette $(CH_2)_m$ steht mit m = 3 oder 4, die durch eine $(C_1-C_2)$-Alkylgruppe an dem C-Atom substituiert ist, das dem N-Atom des Lactamringes benachbart ist, und zwar derart, daß die Konfiguration dieses C-Atoms entgegengesetzt ist zu der des C-4 Atom des Chromansystems.

Ebenfalls ganz besonders bevorzugt sind Verbindungen I, bei denen
$R^1$ für CN oder $SO_2$-$CH_3$ und $R^2$ für H steht,
$R^3$ und $R^4$ gleich oder verschieden sind und für Alkyl mit 1 bis 2 C-Atomen stehen,
X für eine Kette $(CH_2)_m$ steht mit m = 3 oder 4, die durch eine $(C_1-C_2)$-Alkylgruppe an dem C-Atom substituiert ist, das dem N-Atom des Lactamringes benachbart ist, und zwar derart, daß die Konfiguration dieses C-Atoms entgegengesetzt ist zu der des C-4 Atoms im Chromansystem; ebenso bevorzugt sind Verbindungen I, bei denen $R^1$ für $SO_2$- Phenyl, das unsubstituiert oder durch 1 bis 3 Substituenten wie oben erwähnt substituiert ist,
$R^2$ für H oder $OCH_3$ steht,
$R^3$ und $R^4$ gleich sind oder verschieden und für $(C_1-C_2)$-Alkyl stehen,
X für eine Kette $(CH_2)_m$ steht mit m = 3 oder 4, die durch eine $(C_1-C_2)$-Alkylgruppe an dem C-Atom substituiert ist, das dem N-Atom des Lactamringes benachbart ist und zwar derart, daß die Konfiguration dieses C-Atoms entgegengesetzt ist zu der des C-4-Atoms im Chromansystems.

In J. Med. Chem. 1986, 29, 2194-2201 werden Verbindungen beschrieben, die den erfindungsgemäßen Verbindungen am nächsten stehen. Sie sind dort unter folgenden allgemeinen Formeln zusammengefaßt:

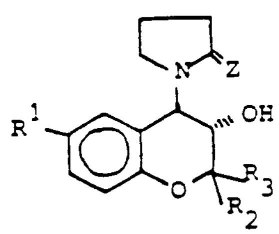

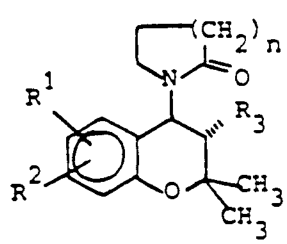

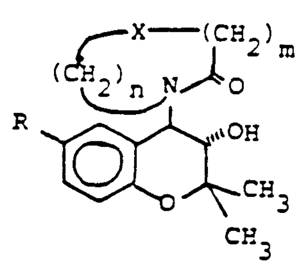

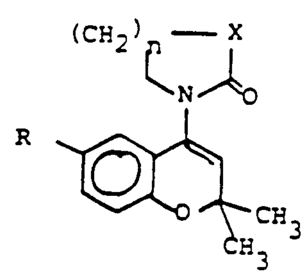

mit $R^1$, $R^2$, $R^3$, Z, n, m, R in den dort aufgeführten Bedeutungen. Ein großer Teil dieser Verbindungen ist auch in verschiedenen Patentanmeldungen beschrieben, zu nennen sind hierbei: EP 107 423, EP 120 427, EP 076 075, EP 120 428, EP-A-173 848 Von diesen Verbindungen ist bekannt, daß sie einen krankhaft erhöhten Blutdruck zu senken vermögen, indem sie die glatte Gefäßmuskulatur relaxieren bzw. gegenüber pressorischen Reizen schützen oder unempfindlich machen können.

Mit den erfindungsgemäßen Verbindungen der Formel I wurde nun eine neue Substanzklasse mit blutdrucksenkenden Eigenschaften gefunden, die sich von den bekannten vor allem dadurch unterscheidet, daß die im Lactamring als Substituent an C-4 des Chromansystems zusätzliche Substituenten in der obengenannten Definition tragen. Diese Substitution führt im Vergleich zu der bereits bekannten unsubstituierten Verbindung zu einer beträchtlichen Steigerung der antihypertensiven Zusatzwirkung. Zusätzlich wurde beobachtet,

daß in einigen Fällen eine solche Wirksteigerung begleitet wird von einer Verminderung der akuten Toxizität, woraus sich insgesamt eine Verbesserung der therapeutischen Breite ableiten läßt. Dies ist gerade bei einer langfristigen Therapie wie die der Hypertoniebehandlung, wo ein Medikament unter Umständen lebenslang eingenommen werden muß, von überragender Bedeutung.

Die sterischen Voraussetzungen, die zu einem solchen vorteilhaften Profil führen, bedürfen der näheren Erläuterung. Es konnte in der oben zitierten Literaturstelle gezeigt werden, daß für eine optimale antihypertensive Wirkung die Substituenten an C-3 und C-4 zueinander in trans-Stellung angeordnet sein müssen. Eine cis-Anordnung führt dagegen zu einer deutlichen Abschwächung der Wirkung. Solcherart transkonfigurierte Verbindungen treten nun zusätzlich noch in Form von Antipoden auf. An Hand eines Beispiels konnte gezeigt werden, daß der (-)-Antipode weitaus stärker wirksam ist als der (+)-Antipode. Dem (-)-Antipoden wurde dabei die 4R, 3S-Konfiguration zugeordnet (vgl. EP 0 120 428).

Durch die Einführung von Substituenten im Lactamring gemäß der Formel I werden nun weitere zusätzliche Asymmetriezentren erzeugt. Die erfindungsgemäßen Verbindungen können dann nicht nur als Antipoden auftreten, sondern man beobachtet zusätzlich das Auftreten von Diastereoisomeren, die bei identischer Konstitutionsformel durchaus mittels gängiger spektroskopischer oder chromatographischer Methoden voneinander unterscheidbar sind. Die Konfiguration eines neuen Asymmetriezentrums im Lactamring kann nun bezüglich etwa der Konfiguration von C-4 im Chromansystem gleich sein oder entgegengesetzt. Ist sie gleich, so ist sie bezüglich der Konfiguration an C-3 entgegengesetzt; ist sie entgegengesetzt, so ist sie bezüglich C-3 gleich. Diese beiden relativen Anordnungen einer bestimmten Konfiguration im Lactamring bezüglich der trans-Konfiguration des Chromansystems manifestieren sich in zwei diastereoisomeren Verbindungen der Formel I. Überraschenderweise werden nun in vielen Fällen die oben erwähnten vorteilhaften antihypertensiven Eigenschaften der Verbindungen I nur für eines der beiden Diastereoisomere beobachtet, während das andere weitaus weniger wirksam ist als die entsprechende unsubstituierte Verbindung. Bevorzugt sind dies solche Verbindungen I, die einen Alkylsubstituenten an dem C-Atom des Lactamrings tragen, das dem Lactamstickstoff benachbart ist und dessen relative Konfiguration mit der des C-4 Atoms im Chromansystems identisch ist.

Die Einführung von Substituenten im Lactamring gemäß der Formel I führt somit nicht, wie man erwarten würde, in allen Fällen zu einer brauchbaren antihypertensiven Wirksamkeit, sondern es ist eine ganz spezielle räumliche Orientierung dieser Substituenten relativ zu den bereits vorhandenen Asymmetriezentren einzuhalten. Einige der Verbindungen der Formel I unterscheiden sich weiterhin von den bereits bekannten Verbindungen auch dadurch, daß sie zusätzlich als Substituenten im Benzoteil einen Arylsulfonyl- oder einem Arylsulfoxy-Substituenten tragen mit Aryl in der eingangs erwähnten Bedeutung. Es war bisher nicht bekannt, daß auch eine solche Substitution zu dieser antihypertensiven Wirksamkeit führen kann.

Weiterhin zeigen einige der erfindungsgemäßen Verbindungen, insbesondere solche Diastereoisomeren, die wie oben erwähnt weniger ausgeprägte Herzkreislaufeffekte haben, einen starken relaxierenden Effekt auf den Ureter. Damit sind solche Verbindungen I wertvolle Therapeutika bei der Behandlung von Harnleiterkoliken oder bei der Lithotripsie-Behandlung. In beiden Fällen erleichtert ein relaxierender Effekt auf den Ureter den Abgang von Steinen.

Die Erfindung betrifft weiterhin ein Verfahren zur Herstellung der Verbindungen I, dadurch gekennzeichnet, daß man

a) Verbindungen der Formel II

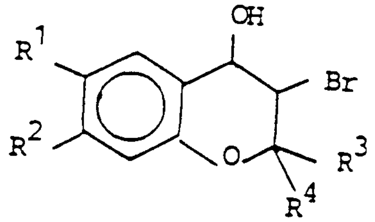

II,

in der $R^1$, $R^2$, $R^3$ und $R^4$ die obengenannten Bedeutungen haben,
umsetzt mit Lactamen der Formel III

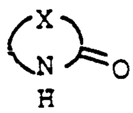

III,

oder

b) Verbindungen der Formel IV

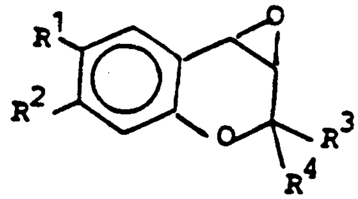

IV,

in der $R^1$, $R^2$, $R^3$ und $R^4$ die obengenannten Bedeutungen haben, umsetzt mit den Lactamen der Formel III

oder

c) Verbindungen der Formel V

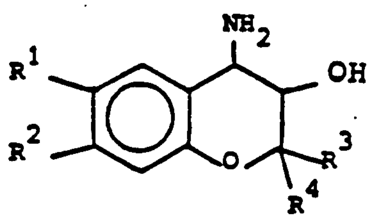

V,

in der $R^1$, $R^2$, $R^3$ und $R^4$ die obengenannten Bedeutungen haben, acyliert zu den Verbindungen VI

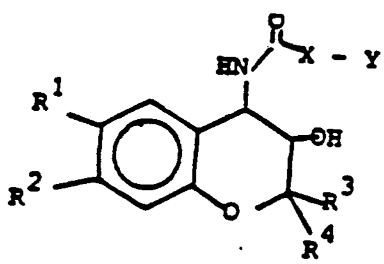

VI,

in denen $R^1$ bis $R^4$ und X wie oben definiert sind und Y die Bedeutung einer Fluchtgruppe, wie Chlor oder Brom, hat,

und diese cyclisiert zu den Verbindungen I,

oder

d) Verbindungen der Formel VII

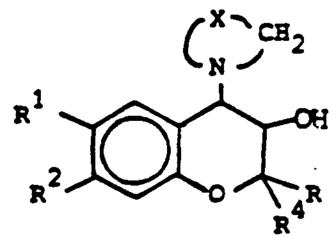

VII,

in der $R^1$ bis $R^4$ und X wie oben definiert sind oxidiert zu den Verbindungen I.

Werden die Verbindungen I nach den Methoden a) oder b) hergestellt, so geschieht dies dadurch, daß man die Verbindungen IV oder II in einem geeigneten Lösemittel, vorzugsweise in dipolar aprotischen Lösemitteln, wie Dimethylsulfoxid oder THF mit den Lactamen III umsetzt, vorzugsweise unter Einwirkung von Basen, wie Natriumhydrid, K-tert.butylat oder ähnlichen, für Lactam-N-Alkylierungen bekanntermaßen geeigneten Basen. Die Reaktionstemperatur ist dabei im weiten Bereich variierbar, vorzugsweise wird zwischen 0° und Zimmertemperatur gearbeitet. Bei Verwendung von racemischen oder auch optisch einheitlichen Lactamen III werden dabei wenigstens 2 neue Produkte der Formel I erhalten. Diese Produkte können durch die üblichen Methoden, wie Kristallisation oder Chromatographie, getrennt werden, in vielen Fällen hat sich auch eine Kombination beider Methoden als günstig erwiesen. Den jeweiligen Produkten kann dann durch gängige physikalische Untersuchungen, wie etwa die Röntgenstrukturanalyse oder die NMR-Spektroskopie, die jeweilige Gesamtkonfiguration zugeordnet werden. Optisch einheitliche, also enantiomerenreine Verbindungen I können durch anschließende Racematspaltung erhalten werden. Werden jedoch bereits enantiomerenreine Lactame III verwendet, so erhält man die diastereoisomeren Verbindungen I ebenfalls in enantiomerenreiner Form und eine Racematspaltung wird überflüssig.

Lactame der Formel III sind in vielen Fällen bekannt oder können leicht nach literaturbekannten Methoden hergestellt werden. Ebenso bekannt sind in vielen Fällen die Bromhydrine II bzw. die Epoxide IV (vgl. hierzu die oben zitierten Patentschriften oder J. Med. Chem. 1986,29, 2194-2201) oder können analog nach dort angegebenen Methoden hergestellt werden. Bislang nicht bekannt sind solche Verbindungen II und IV, bei denen $R^1$ in der Bedeutung $-SO_n-Ar$ steht mit n und Ar in den obengenannten Bedeutungen. Sie können z.B. nach folgendem Schema hergestellt werden:
2,3-Dihydro-2H-benzo[b]pyran-4-one der Formel VIII

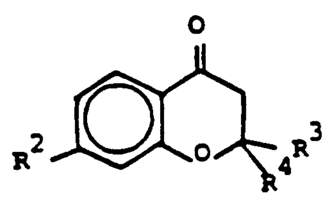

VIII

werden mit Säurechloriden $Ar-SO_2-Cl$
in an sich bekannter Weise nach Art der Friedel-Crafts Acylierung umgesetzt zu Verbindungen der Formel IX

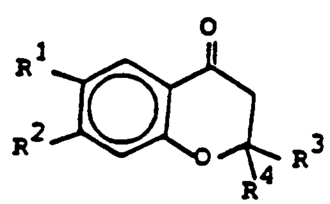

IX,

in denen $R^1$ für $ArSO_n$ (mit Ar und n wie oben definiert) steht und in denen $R^2$, $R^3$ und $R^4$ wie oben definiert sind. Diese Verbindungen IX werden durch Reduktionen unter Standardbedingungen, etwa durch $NaBH_4$ in Methanol, umgesetzt zu den Verbindungen X

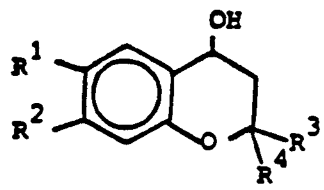

X

und anschließend einer Wasserabspaltung, etwa durch Pyridin/Phosphoroxychlorid unterworfen, wobei 2H-Benzo[b]pyrene der Formel XI entstehen:

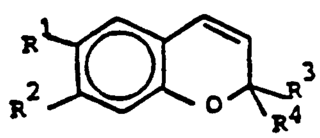

XI.

Verbindungen XI lassen sich leicht nach Standard-Verfahren in die Epoxide IV oder die Bromhydrine II umwandeln. Steht bei dieser Reaktionssequenz $R^2$ in der Bedeutung von $NH_2$ oder OH, so sind gegebenenfalls Schutzgruppen, wie etwa die Dimethylaminomethylengruppe für $NH_2$ oder die Acetyl- oder Methylgruppe für die OH-Gruppe, notwendig. Diese werden auf geeigneten Stufen, vorzugsweise nach Durchführung der in Verfahren a) oder b) beschriebenen Umsetzungen durch gängige Methoden wieder abgespaltet.

Die erfindungsgemäße Verbindungen der Formel I sind, wie bereits erwähnt, Antihypertensiva, die als Pharmazeutika in der Human- und Veterinärmedizin eingesetzt werden können. Sie werden in Dosierungen von mindestens 0,001 mg/kg/Tag, vorzugsweise 0,005 mg und insbesondere 0,55 mg/kg/Tag bis maximal 10 mg/kg/Tag, vorzugsweise 5 mg/kg/Tag und insbesondere 2 mg/kg/Tag in Kapseln, Dragees, Tabletten, Puder, Zäpfchen oder Lösungen mit Zusätzen oder ohne Zusätze von galenischen Hilfsstoffen enteral, z.B. oral oder parenteral (wie etwa durch Injektion in das Gefäßsystem, z.B. intravenös) verabfolgt, jeweils bezogen auf ein Gewicht von ca. 75 kg. Sie eignen sich zur Behandlung der Hypertonie, alleine oder in Kombination mit anderen antihypertensiv wirkenden Arzneimitteln, wie etwa Diuretika, Ca-Antagonisten oder ACE-Hemmer.

Diese Angaben beziehen sich auf einen Menschen vom Gewicht von 75 kg.

**Beispiel 1**

(3R*, 4S*, 5'S*)-6-Cyano-3,4-dihydro-2,2-dimethyl-4-[2'-oxo-5'-methyl-1-pyrrolidinyl)-2H-benzo[b]pyran-3-ol und (3R*, 4S*, 5'R*)-6-Cyano-3,4-dihydro-2,2-dimethyl-4-[2'-oxo-5'-methyl-1-pyrrolidinyl)-2H-benzo[b]pyran-3-ol.

Zu einer Lösung von 32 g (0,113 Mol) 6-Cyano-trans-3-bromo-3,4-dihydro-2,2-dimethyl-2H-benzo[b]-pyran-4-ol in 200 ml DMSO werden bei 20° 3,6 g (0,12 Mol) 80 %iges NaH (Suspension in Öl) eingetragen. Nach einstündigem Rühren werden weitere 4,8 g (0.16 Mol) 80 %iges NaH und 15,9 g (0,16 Mol) racemisches 5-Methyl-2-pyrrolidon eingetragen. Nach 8-stündigem Rühren bei 20° wird das Reaktionsgemisch in Eiswasser gegossen und der Niederschlag aus Methanol umkristallisiert.

Das umkristallisierte Gemisch wird über eine Kieselgelsäule mit $CH_2Cl_2$ / $CH_3OH$ (95:5) aufgetrennt. (3R*, 4S*, 5'S*)-6-Cyano-3,4-dihydro-2,2-dimethyl-4-[2'-oxo-5'-methyl-1'-pyrrolidinyl]-2H-benzo[b]pyran-3-ol wird isoliert und mehrfach aus Ethanol umkristallisiert.

Kristalle vom Schmp: 239-240°

NMR ($d_6$-DMSO) $CH_3$-Signale $\delta$ 0,80 (3H, d)
1,17 (3H, s)
1,45 (3H, s)
Analyse: berechnet für $C_{17}H_{20}N_2O_3$ : 300,37
Ber. C, 67,98; H, 6,71; N, 9,33 %
Gef. C, 67,9; H, 6,8; N,9,4 %

Die methanolische Mutterlauge, in der (3R*, 4S*, 5'R*)-6-Cyano-3,4-dihydro-2,2-dimethyl-4-[2'-oxo-5'-methyl-1'-pyrrolidinyl)-2H-benzo[b]pyran-3-ol angereichert ist, wird eingedampft und die Verbindung über eine Kieselgelsäule mit $CH_2Cl_2$ / $CH_3OH$ (95:5) isoliert.

Kristalle vom Schmp: 190-192° (aus Essigester)
NMR ($d_6$-DMSO) $CH_3$-Signale δ 1,20 (3H, s)
1,30 (1,5H, s)
1,43 (4,5H, s)
Analyse: berechnet für $C_{17}H_{20}N_2O_3$ : 300,37
Ber. C, 67,98; H, 6,71; N, 9,33 %
Gef. C, 68,0; H, 6,7; N, 9,4 %

**Beispiel 2**

(-)-(3S, 4R, 5'R)-6-Cyano-3,4-dihydro-2,2-dimethyl-4-(2'-oxo-5'-methyl-1-pyrrolidinyl)-2H-benzo[b]pyran-3-ol und (-)-(3R, 4S,5'R)-6-Cyano-3,4-dihydro-2,2-dimethyl-4-)2'-oxo-5'-methyl-1-pyrrolidinyl)-2H-benzo[b]pyran-3-ol

Zu einer Lösung von 30,2 g (0,15 Mol) 6-Cyano-3,4-dihydro-2,2-dimthyl-3,4epoxy-2H-benzo[b]pyran in 150 ml Dimethylsulfoxid werden 6 g (0,2 Mol) 80 %iges NaH und 15 g (0,15 Mol) (R)-5-Methyl-2-pyrrolidon (s. René Amstutz, Björn Ringdahl, Bo Karlén, Margareth Rock und Donald J. Jenden, J. Med. Chem. 1985, 28, 1760-1765) eingetragen und 6 Stunden bei 20° gerührt. Das Reaktionsgemisch wird in Eiswasser eingetragen, der Niederschlag neutral gewaschen, getrocknet und über eine Kieselgelsäule mit $CH_2Cl_2$ / $CH_3OH$ 19:1 getrennt und aus Ethanol umkristallisiert. Man erhält zunächst das
(-)-(3R, 4S, 5'R)-6-Cyano-3,4-dihydro-2,2-dimethyl-4-(2'-oxo-5'-methyl-1'-pyrrolidinyl)-2H-benzo[b]pyran-3-ol
Kristalle von Schmp.: 184° $^{20}_{\alpha D}$ = -27,8° c = 1 in Ethanol.

Als 2. langsamer laufendes Produkt erhält man nach zusätzlicher Kristallisation aus Ethanol das (-)-(3S,4R,5'R)-6-Cyano-3,4-dihydro-2,2-dimethyl-4-(2'-oxo-5'-methyl-1-pyrolidinyl)-2H-benzo[b]pyran-3-ol.
Kristalle vom Schmp.: 258° $\alpha_D^{20}$ = -86° (c = 1, Ethanol)

**Beispiel 3**

6-Cyano-3,4-dihydro-2,2-dimethyl-trans-4-(2'-oxo-3'-methyl-1-pyrrolidinyl)-2H-benzo[b]pyran-3-ol

Diastereomer A und Diastereomer B

Diastereomer B:

Zu einer Lösung von 16 g (0,056 Mol) 6-Cyano-trans-3-bromo-3,4-dihydro-2,2-dihydro-2H-benzo[b]-pyran-4-ol in 80 ml Dimethylsulfoxid werden 1,8 g (0,06 Mol) 80 %iges NaH zugefügt. Nach einstündigem Rühren bei 20° werden weitere 2,4 g (0,08 Mol) 80 %iges NaH und 8,6 g (0,084 Mol) racemisches 3-Methyl-2-pyrrolidon zugefügt, und dann wird 16 Stunden bei 20° gerührt. Nach dem Abkühlen und Eintropfen von 160 ml Wasser wird der Niederschlag abgesaugt, mit wenig Wasser gewaschen und getrocknet.

Das Rohprodukt wird zweimal aus Essigester/Methanol und anschließend aus Dimethylformamid/Methanol umkristallisiert. Das Diastereomer läßt sich so rein isolieren.
Kristalle vom Schmp: 261-263°
NMR ($d_6$-DMSO) $CH_3$-Signale: δ 1,19 (3H, s)
1,21 (6H, s)
1,45 (3H, s)

Diastereomer A:

Die Mutterlaugen aus dem obigen Versuch werden eingedampft und der Rückstand wird über eine Kieselgelsäule mit Essigester/Petrolether (9:1) chromatographiert. Das Diastereomer A wird abgetrennt und aus Ethanol umkristallisiert.
Kristalle vom Schmp: 210-211°
NMR ($d_6$-DMSO) $CH_3$-Signale: δ 1,13 (3H, d)
1,20 (3H, s)
1,47 (3H, s)

**Beispiel 4**

(3R*, 4S*, 5′S*)-3,4-Dihydro-2,2-dimethyl-6-methylsulfonyl-4-(2′-oxo-5′-methyl-1-pyrrolidinyl)-2H-benzo[b]-pyran-3-ol

Zu einer Lösung von 10,2 g (0,04 Mol) 3,4-Dihydro-2,2-dimethyl-3,4-epoxi-6-methylsulfonyl-2H-benzo-[b]pyran und 4 g (0,04 Mol) rac. 5-Methyl-2-pyrrolidon in 50 ml DMSO werden bei 20° 1,5 g (0,05 Mol) 80 %iges NaH eingetragen. Man erhitzt 30 Minuten auf 45°C, läßt 5 Std. bei 20° rühren und gießt in Eiswasser ein. Es wird mit Aktivkohle geklärt und das Produkt ausgesalzen. Der Niederschlag wird abgesaugt und das Diastereomer A wird über eine Kieselgelsäule mit Ethanol/Essigester/Toluol/Petrolether 2:2:1:1 abgetrennt und aus Essigester umkristallisiert.
Kristalle vom Schmp: 190-193°
NMR ($d_6$-DMSO) $CH_3$-Signale: $\delta$ 0,74 (3H, d)
1,18 (3H, s)
1,47 (3H, s)

Herstellung des Ausgangsmaterials:

p-Methylsulfonylphenyl-1,1-dimethylpropargylether erhält man aus p-Methylsulfonylphenol und 3-Methyl-3-chlorbutin in an sich bekannter Weise.
Schmp.: 64-65° (aus wenig Petrolether)
2,2-Dimethyl-6-methylsulfonylchromen erhält man durch Erhitzen des p-Methylsulfonylphenyl-1,1-dimethylpropargylethers in 1,2-Dichlorobenzol auf 180°
Schmp.: 107-108° (aus Diisopropylether)
3-Brom-3,4-dihydro-2,2-dimethyl-6-methylsulfonyl-2H-benzo [b]pyran-4-ol erhält man aus 2,2-Dimethyl-6-methylsulfonylchromen und N-Bromsuccinimid in einem 9:1 Dimethylsulfoxid/$H_2O$ Gemisch
Schmp.: 140-141° aus Diisopropylether
3,4-Dihydro-2,2-dimethyl-3,4-epoxi-6-methylsulfonyl-2H-benzo[b]pyran erhält man durch Umsatz von 3-Brom-3,4-dihydro-2,2-dimethyl-6-methylsulfonyl-2H-benzo[b]pyran-4-ol mit Natriumhydrid in DMSO.
Schmp.: 165° (aus Essigester)

**Beispiel 5**

(3R*, 4S*, 5′S*)-2,2-Dimethyl-7-methoxy-6-phenylsulfonyl-4-(2′-oxo-5′-methyl-1′-pyrrolidinyl)-2H-benzo[b]-pyran-3-ol und (3R*, 4S*, 5′R*)-2,2-Dimethyl-7-methoxy-6-phenylsulfonyl-4-(2′-oxo-5′-methyl-1′-pyrrolidinyl)-2H-benzo[b]pyran-3-ol

Zu einer Lösung von 9,6 g 3-Brom-3,4-dihydro-2,2-dimethyl-7-methoxy-6-phenylsulfonyl-2H-benzo[b]-pyran-3-ol in 60 ml Dimethylsulfoxid gibt man 1,62 g (0,0675 Mol) NaH in Form einer 80 %igen Suspension sowie 10,3 g (0,11 Mol) (±)-5-Methyl-2-pyrrolidinon und rührt die Mischung 4 h bei 40°. Anschließend gibt man die Mischung auf Eiswasser und saugt ab. Der Rückstand wird neutral gewaschen, getrocknet und der Chromatographie an Kieselgel mit dem Elutionsmittel Toluol/Methylenchlorid/Methanol 10:1:1 unterworfen. Als erstes Hauptprodukt wird dabei das (3R*, 4S*, 5′R*)-2,2-dimethyl-7-methoxy-6-phenylsulfonyl-4-(2′-oxo-5′-methyl-1′-pyrrolidinyl)-2H-benzo[b]pyran-3-ol eluiert: Schmp.: 214 - 15°C; NMR ($d_6$-DMSO) $\delta$ (ppm): 1,17; 1,27; 1,4 (s,s,s; 9H, 2,2-Dimethyl und 5-Methyl)
Als zweites Hauptprodukt erhält man das langsamer laufende (3R*, 4S*, 5′S*)-2,2-Dimethyl-7-methoxy-6-phenylsulfonyl-4-(2′-oxo-5′-methyl-1-pyrrolidinyl)-2H-benzo[b]pyran-3-ol Schmp.: 244 - 245°C; NMR ($d_6$-DMSO) $\delta$ (ppm): 0,73 (d, 3H, 5′-$CH_3$)

Herstellung des Ausgangsmaterials:

3-Brom-3,4-dihydro-2,2-dimethyl-7-methoxy-6-phenylsulfonyl-2H-benzo[b]pyran-4-ol erhält man aus 2,2-Dimethyl-7-methoxy-6-phenylsulfonyl-2H-chromen und N-Bromsuccinimid in einem 9:1 Dimethylsulfoxid/$H_2O$ Gemisch. Schmp.: 202 - 203°C.

2,2-Dimethyl-7-methoxy-6-phenylsulfonyl-2H-chromen erhält man aus 2,2-Dimethyl-4-hydroxy-7-methoxy-6-phenylsulfonylchroman mit Pyridin/Phosphoroxychlorid in Benzol.
Schmp.: 140 - 141°C
2,2-Dimethyl-4-hydroxy-7-methoxy-6-phenylsulfonylchroman erhält man aus 2,2-Dimethyl-7-methoxy-6-phenylsulfonylchroman-4-on mit Natriumborhydrid in Methanol.
Schmp.: 146 - 147 °C
2,2-Dimethyl-7-methoxy-6-phenylsulfonylchroman-4-on erhält man aus Phenylsulfonylchlorid, 2,2-Dimethyl-7-methoxychroman-4-on und Aluminiumchlorid in Methylenchlorid.
Schmp.: 223 - 225 °C.

**Beispiel 6**

(3R*, 4S*, 5′S*)-3,4-Dihydro-2,2-dimethyl-6-phenylsulfonyl-4-[2′-oxo-5′-methyl-1-pyrrolidinyl-2H-benzo[b]-pyran-3-ol und 3R*, 4S*, 5′R*-3,4-Dihydro-2,2-dimethyl-6-phenylsulfonyl-4-(2′-oxo-5′-methyl-1-pyrrolidinyl)-2H- benzo[b]pyran-3-ol

Die Verbindungen werden analog Beispiel 5 mit rac. 5-Methylpyrrolidin-2-on hergestellt. Anschließend wird das Gemisch auf einer Kieselgelsäule mit Methylenchlorid/Essigester/Ethanol (94:3:3) aufgetrennt.
(3R*, 4S*, 5′S*)-3,4-Dihydro-2,2-dimethyl-6-phenylsulfonyl-4-(2′-oxo-5′-methyl-1-pyrrolidinyl)-2H-benzo-[b]pyran-3-ol
Kristalle vom Schmp: 216-217 ° aus Ethanol
NMR ($d_6$ -DMSO) 5′-$CH_3$-Signal: δ 0.48 (3H, Pseudoduplett)
(3R*, 4S*, 5′R*)-3,4-Dihydro-2,2-dimethyl-6-phenylsulfonyl-4-(2′-oxo-5′-methyl-1-pyrrolidinyl)-2H-benzo[b]-pyran-3-ol
Kristalle vom Schmp: 240-241 °C aus DMF/Ethanol
NMR ($d_6$-DMSO) 5′-$CH_3$-Signal: δ 1,27 (3H, d)
Erfindungsgemäß können außer den in den Ausführungsbeispielen beschriebenen Verbindungen auch die in der folgenden Tabelle zusammengestellten Verbindungen der Formel I erhalten werden:

1. (3R, 4S, 5′R)-3,4-Dihydro-2,2-dimethyl-6-methylsulfonyl-4-[2′-oxo-5′-methyl-1-pyrrolidinyl)-2-H-benzo-[b]pyran-3-ol
2. (3R*, 4S*, 5′S*) -6-Cyano-3,4-dihydro-2,2-dimethyl-7-methoxy-4-[2′-oxo-5′-methyl-1-pyrrolidinyl)-2H-benzo[b]pyran-3-ol,
3. (3R*, 4S*, 4′S*)-6-Cyano-3,4-dihydro-2,2-dimethyl-7-hydroxy-4-[2′-oxo-4′-methyl-1-pyrrolidinyl)-2H-benzo[b] pyran-3-ol,
4. (3R*, 4S*, 3′R*)-3,4-Dihydro-2,2-diethyl-6-nitro-4[2′-oxo-3′-methyl-1-pyrrolidinyl)-2H-benzo[b]pyran-3-ol,
5. (3R, 4S, 5'S)-7-amino-3,4-dihydro-2,2-dimethyl-6-nitro-4-[2'-oxo-5'-methyl-1-pyrrolidinyl)-2H-benzo[b] pyran-3-ol,
6. (3R*, 4S*, 5'S*)-3,4-dihydro-2-methyl-2-ethyl-6-phenylsulfonyl-4-[2'-oxo-5'-methyl-1-pyrrolidinyl)-2H-benzo [b]pyran-3-ol,
7. (3R*, 4S*, 3'S*)-3,4-Dihydro-2,2-diethyl-6-[p-nitro]-phenylsulfonyl-4-[2'-oxo-3'-methyl-1-pyrrolidinyl]-2H-benzo[b]pyran-3-ol,
8. (3R*, 4S*, 3'R*, 4'S*)-3,4-Dihydro-2,2,-dimethyl-6-[p-cyano]-phenylsulfonyl-4-[2'-oxo-3'-methyl-4'-methyl-1-pyrrolidinyl]-2H-benzo[b]pyran-3-ol,
9. (3R*, 4S*, 5'S*)-3,4-Dihydro-2,2-dimethyl-6-[p-chlor]-phenylsulfonyl-4-[2'-oxo-5'-methyl-1-pyrrolidinyl]-2H-benzo[b]pyran-3-ol,
10. (3R, 4S, 3'R, 4'S)-3,4-Dihydro-2-methyl-2-propyl)-6[p-methoxy]phenylsulfonyl-4-[2'-oxo-3'-methyl-4'-ethyl-1-pyrrolidinyl]-2H-benzo[b]pyran-3-ol,
11. (3R*, 4S*, 4'R*)-3,4-Dihydro-2,2,-dimethyl-6-phenylsulfonyl-4-[2'-oxo-4'-methyl-1-pyrrolidinyl]-2H-benzo [b]pyran-3-ol,
12. (3R, 4S, 5'S)-3,4-dihydro-2,2-Diethyl-6-[p-nitro]-phenylsulfonyl-4-[2'-oxo-5'-methyl-1-pyrrolidinyl]-2H-benzo[b]pyran-3-ol,
13. (3R*, 4S*, 3'R*)-3,4-Dihydro-2,2-dimthyl-6-[p-cyano]-phenylsulfonyl-4-[2'-oxo-3'-methyl-1-pyrrolidinyl]-2H-benzo[b]pyran-3-ol,
14. (3R*, 4S*, 5'S*)-3,4-Dihydro-2,2,-dimethyl-6-[p-chlor] phenylsulfonyl-4-[2'-oxo-5'-ethyl-1-pyrrolidinyl]-2H-benzo[b]pyran-3-ol,
15. (3R*, 4S*, 5'S*)-3,4-Dihydro-2,2-dimethyl-6-phenylsulfonyl-4-[2'-oxo-5'-methyl-1-morpholinyl]-2H-benzo[b] pyran-3-ol,
16. (3R*, 4S*, 6'S*)-6-Cyano-3,4-dihydro-2,2,-dimethyl-4-[2'-oxo-6'-methyl-1-piperazinyl)-2H-benzo[b]-pyran-3-ol,
17. (3R*, 4S*, 2'S*)-6-Cyano-3,4-dihydro-2,2-dimethyl-4-[4'-oxo-2'-methyl-3-thiazolidinyl)-2H-benzo[b]-pyran-3-ol,

18. (3R*, 4S*, 6'S*)-6-Cyano-3,4-dihydro-2,2-diethyl-4-[2'-oxo-6'-methyl-1-piperidinyl)-2H-benzo[b]pyran-3-ol,

**Pharmakologische Daten**

Der Einfluß der Verbindungen auf den arteriellen Blutdruck wurde an narkotisierten normotensiven männlichen Sprague-Dawley Ratten untersucht. Als Betäubungsmittel wurde Nembutal® in einer Dosis von 70 mg/kg i.p. verwendet. Die Blutdruckmessung erfolgte mittels Katheter in der Arteria carotis mit einem Druckwandler (Statham P 23 Db). Für die i.v. Applikation wurden die Substanzen in Dimethylsulfoxid gelöst und mit Wasser verdünnt. Die Konzentration der Stammlösung war 1mg/ml, wobei die Lösung 15 % Dimethylsulfoxid enthielt. Anteile dieser Stammlösung wurden für die Injektion jeweils frisch mit Wasser verdünnt.

Es kamen Konzentrationen von 50 (100), 30 (30) und 10 (10) µg/ml für die i.v. Applikation, 100 (100), 50 (60) und 30 (30) µg/ml für die i.d. Applikation zur Anwendung. Die angegebenen Zahlen beziehen sich dabei auf das Experiment a), die in Klammern angegebenen Zahlen auf Experiment b).

Die Blutdruckmessung wurde nach 0; 0,2; 0,4; 1; 2; 3; 10; 15 und 30 min. nach der i.v. Applikation sowie nach 0; 1; 3; 5; 10; 15; 20; 30; 40; 50 und 60 min. nach der i.d. Applikation vorgenommen. Die $ED_{25}$ Werte wurden aus dem Minima der jeweiligen Blutdruckkurven bestimmt.

a) (±)-(3R*, 4S*, 5'S*)-6-Cyano-3,4-dihydro-2,2-dimethyl-4-(2'-oxo-5'-methyl-1'-pyrrolidinyl)-2H-benzo[b]-pyran-3-ol
(Verbindung aus Beispiel 1):
$ED_{25}$ = 19 µg/kg (i.v.) $ED_{25}$ = 42 µg/kg (i.d.)
b) (±)-6-Cyano-3,4-dihydro-2,2-dimethyl-trans-4-(2-oxo-1-pyrrolidinyl-2H-benzo[b]pyran-3-ol
(Beispiel aus J. Med. Chem. 1986, 29, 2194-2201)
$ED_{25}$ = 42 µg/kg (i.v.) $ED_{25}$ = 82 µg/kg (i.d.)

**Beeinflussung der KCl-induzierten rhythmischen Kontraktionen an Meerschweinchen-Ureteren in vitro**

Methode:

Männliche Meerschweinchen wurden durch Nackenschlag und Entbluten aus den Carotiden getötet. Die beiden Ureteren wurden sofort entnommen, wobei der Bereich nahe dem Nierenkelch wegen der dort vorhandenen Schrittmacher-Aktivität gemieden wurde. 2 cm lange Stücke wurden zunächst in einer Petrischale, die Tyrode-Lösung enthielt, von Bindegewebe befreit und dann jeweils mit einer Vorspannung von 4,9 mN (=0,5 p) in ein 25 ml-Organbad (Fa. Rhema Labortechnik, Hofheim) gehängt. Das Organbad enthielt eine auf 37° gehaltene und mit Carbogen (95 % $O_2$, 5 % $CO_2$) durchperlte Tyrodelösung mit folgender Zusammensetzung (mmol/l): NaCl 137, KCl 2,68, $MgSO_4$ 1,05, $CaCl_2$ 1,8, $NaH_2PO_4$ 0,41, $NaHCO_3$ 11,9, Glucose 5,55.
Die Kontraktionen wurden isometrisch gemessen unter Verwendung von Gould/Statham UC2 Gebern. Nach einer Äquiilibrierungszeit von mindestens 15 Minuten wurde KCl in das Organbad zugegeben, wobei eine Konzentration von 4 x $10^{-2}$ mol/l erreicht wurde. Der Agonist wurde 2 Minuten im Bad belassen, wobei phasische Kontraktionen auftraten, ohne einen nennenswerten Anstieg der Grundspannung zu bewirken.

Danach wurde 1 Minute ausgespült, woraufhin die rhythmischen Kontraktionen sofort aufhörten. Nach einem zweiten Durchgang von Agonistgabe und Spülvorgang wurde die Prüfsubstanz (Benzopyran-Derivat) in das Organbad zugegeben (in Form einer Lösung in 0,1 ml Ethanol, die Endkonzentrationen betrugen in allen Fällen $10^{-7}$ mol/l) und eine Einwirkungszeit von einer Minute abgewartet, bevor KCl zugesetzt wurde. An den folgenden Spülvorgang schlossen sich zwei abschließende Agonistgabe/Spülgänge an. Von den jeweils zweiminütigen Perioden unter KCl-Einwirkung wurden folgende Parameter bestimmt: 1. mittlere Kontraktionskraft, 2. Frequenz der Kontraktionen und 3. Produkt aus mittlerer Kontraktionskraft und Frequenz.
Ausschlußkriterien waren mittlere Kontraktionskräfte unter 4 mN oder Frequenzen unter 2/min bei mehr als einer der vier Perioden, bei denen nur der Agonist KCl im Bad vorhanden war. Ausgewertet wurde die prozentuale Hemmung unter Prüfsubstanz im Vergleich zum gemittelten Wert aus den beiden Vorläufen.

Neben dem arithmetischen Mittelwert (x) wurde der Standardfehler des Mittelwerts (SEM) errechnet.

Ergebnisse:

| Verbindung | mittlere Kontraktionskraft k [%] | Frequenz v [%] | k·v [%] | n |
|---|---|---|---|---|
| (-)-(3R,4S,5'R)-6-Cyano-3,4-dihydro-2,2-dimethyl-4-(2'-oxo-5'-methyl-1'-pyrrolidinyl)-2H-benzo[b]pyran-3-ol aus Beispiel Nr. 2 | 75 ± 8 | 89 ± 4 | 96 ± 2 | 7 |

| Verbindung | mittlere Kontraktionskraft k [%] | Frequenz v [%] | k·v [%] | n |
|---|---|---|---|---|
| ±)-(3R*,4S*,5'R*)-6-Cyano-3,4-dihydro-2,2-dimethyl-4-(2'-oxo-5-methyl)-1-pyrrolidinyl)-2H-benzo[b]pyran-3-ol aus Beispiel Nr. 1 | 69 ± 11 | 86 ± 4 | 96 ± 1 | 6 |
| ±)-6-Cyano-3,4-dihydro-2,2-dimethyl-trans-4-(2-oxo-1-pyrrolidinyl-2H-benzo[b]-pyran-3-ol (aus J.Med.Chem. 1986,29 2194-2201) | 8 ± 8 | 58 ± 5 | 61 ± 7 | 7 |

n = Anzahl der Ureteren

Literatur:

P. SCHIANTARELLI und W. MURMANN.
Antispadmodic Activity of Rociverine on the Smooth Musculature of the Urinary Tract.
Arzneim.-Forsch./Drug Res. 30, 1102-1109 (1980)

**Patentansprüche**

**Patentansprüche für folgende Vertragsstaaten : AT, BE, CH, DE, FR, GB, IT, LI, LU, NL, SE**

1. N-Benzopyranyllactame der Formel I

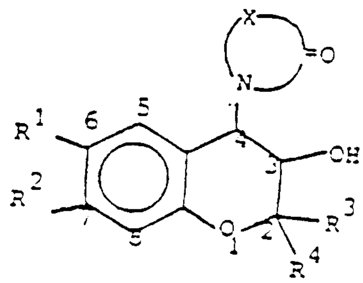

I,

in welcher

$R^1$ für CN, $NO_2$, $SO_n$-($C_1$-$C_6$)-Alkyl, $SO_n$-Ar, steht; wobei n = 1 oder 2, Ar für Phenyl steht, das unsubstituiert oder durch 1 bis 3 gleiche oder verschiedene Reste ($C_1$-$C_2$)-Alkyl, ($C_1$-$C_2$)Alkoxy, Halogen, Trifluormethyl, CN, $NO_2$, CO-($C_1$-$C_2$)-Alkyl, $SO_p$-($C_1$-$C_2$)-Alkyl, substituiert ist und p für 1 oder 2 steht,

$R^2$ für H, OH, ($C_1$-$C_2$)-Alkoxy, ($C_1$-$C_2$)-Alkyl, Halogen oder $NR^5R^6$ steht, wobei $R^5$ und $R^6$ gleich oder verschieden sind und für H, ($C_1$-$C_2$)Alkyl oder ($C_1$-$C_2$)-Alkylcarbonyl stehen,

$R^3$ und $R^4$ gleich oder verschieden sind und für Alkyl mit 1-4 C-Atomen stehen,

die oben genannten Bedeutungen von $R^1$ und $R^2$ auch vertauscht sein können,

X für eine Kette $(CH_2)_m$ steht, die durch mindestens 1 und höchstens 2m-1 ($C_1$-$C_2$)-Alkylgruppen substituiert ist, sowie durch ein Heteroatom Y in der Bedeutung von O, $NR^7$, S unterbrochen sein kann und $R^7$ H oder ($C_1$-$C_4$)-Alkyl bedeutet und m für 2, 3 oder 4 steht, wobei die Konfiguration an $C_3$ und $C_4$ stets entgegengesetzt ist.

2. Verbindung I nach Anspruch 1, dadurch gekennzeichnet, daß mindestens einer der Substituenten oder Indices folgende Bedeutung hat:
$R^1$, $R^2$, $R^3$, $R^4$ wie in Anspruch 1 definiert,
X eine Kette $(CH_2)_m$, die durch eine ($C_1$-$C_2$)-Alkylgruppe substituiert ist, sowie durch ein Heteroatom Y, das für O, S oder $NR^7$ steht mit $R^7$ in der Bedeutung von H oder ($C_2$-$C_4$)-Alkyl, unterbrochen sein kann und wobei m für 2, 3 oder 4 steht.

3. Verbindung I nach Anspruch 1, dadurch gekennzeichnet, daß mindestens einer der Substituenten oder Indices folgende Bedeutung hat:
$R^1$ bis $R^4$ wie in Anspruch 1 definiert,
X eine Kette $(CH_2)_m$, die durch eine ($C_1$-$C_2$)-Alkylgruppe substituiert ist wobei m für 3 oder 4 steht.

4. Verbindung I nach Anspruch 1, dadurch gekennzeichnet, daß mindestens einer der Substituenten oder Indices folgende Bedeutung hat:
$R^1$ bis $R^4$ wie in Anspruch 1 definiert,
X eine Kette $(CH_2)_m$, wobei m für 3 oder 4 steht, die durch eine ($C_1$-$C_2$)-Alkylgruppe an dem C-Atom substituiert ist, das dem N-Atom des Lactamringes benachbart ist.

5. Verbindung I nach Anspruch 1, dadurch gekennzeichnet, daß mindestens einer der Substituenten oder Indices folgende Bedeutung hat:
$R^1$-$R^4$ wie in Anspruch 1 definiert,
X eine Kette $(CH_2)_m$ mit m = 3 oder 4, die durch eine ($C_1$-$C_2$)-Alkylgruppe an dem C-Atom substituiert ist, das dem N-Atom des Lactamringes benachbart ist, wobei die Konfiguration dieses C-Atoms dieselbe ist wie die des C-4 Atoms des Chromansystems.

6. Verbindung I nach Anspruch 1, dadurch gekennzeichnet, daß mindestens einer der Substituenten oder Indices folgende Bedeutung hat:

$R^1$-$R^4$ wie in Anspruch 1 definiert,
X eine Kette $(CH_2)_m$ mit m = 3 oder 4, die durch eine $(C_1\text{-}C_2)$-Alkylgruppe an dem C-Atom substituiert ist, das dem N-Atom des Lactamringes benachbart ist, wobei die Konfiguration dieses C-Atoms entgegengesetzt ist zu der des C-4 Atoms des Chromansystems.

**7.** Verbindung I nach Anspruch 1, dadurch gekennzeichnet, daß mindestens einer der Substituenten oder Indices folgende Bedeutung hat:

| | |
|---|---|
| $R^1$ | CN, $SO_2CH_3$ |
| $R^2$ | H, |
| $R^3$ und $R^4$ | (gleich oder verschieden) $(C_1\text{-}C_2)$-Alkyl, |
| X | eine Kette $(CH_2)_m$ mit m = 3 oder 4, die durch eine $(C_1\text{-}C_2)$-Alkylgruppe an dem C-Atom substituiert ist, das dem N-Atom des Lactamringes benachbart ist, wobei die Konfiguration dieses C-Atoms dieselbe ist wie das des C-4 Atoms im Chromansystem. |

**8.** Verbindung I nach Anspruch 1, dadurch gekennzeichnet, daß mindestens einer der Substituenten oder Indices folgende Bedeutung hat:

| | |
|---|---|
| $R^1$ | CN, $SO_2CH_3$ |
| $R^2$ | H, |
| $R^3$ und $R^4$ | (gleich oder verschieden) $(C_1\text{-}C_2)$-Alkyl, |
| X | eine Kette $(CH_2)_m$ mit m = 3 oder 4, die durch eine $(C_1\text{-}C_2)$-Alkylgruppe an dem C-Atom substituiert ist, das dem N-Atom des Lactamringes benachbart ist, wobei die Konfiguration dieses C-Atoms entgegengesetzt ist zu der des C-4 Atoms im Chromansystem. |

**9.** Verbindung I nach Anspruch 1, dadurch gekennzeichnet, daß mindestens einer der Substituenten oder Indices folgende Bedeutung hat:

| | |
|---|---|
| $R^1$ | $SO_2Ar$ mit Ar gleich Phenyl, das unsubstituiert oder wie in Anspruch 1 definiert substituiert ist, |
| $R^2$ | H, $CH_3$, |
| $R^3$ und $R^4$ | (gleich oder verschieden) $(C_1\text{-}C_2)$-Alkyl, |
| X | $(CH_2)_m$ mit m = 3 oder 4, substituiert durch eine $(C_1\text{-}C_2)$-Alkylgruppe an dem C-Atom, das dem N-Atom des Lactamringes benachbart ist, wobei die Konfiguration dieses C-Atoms dieselbe ist wie die des C-4-Atoms im Chromansystem. |

**10.** Verbindung nach Anspruch 1, dadurch gekennzeichnet, daß sie (-)-(3R, 4S, 5'R)-6-Cyano-3,4-dihydro-2,2-dimethyl-4-(2'-oxo-5'-methyl-1-pyrrolidinyl)-2H-benzo[b]pyran-3-ol ist.

**11.** Verbindung nach Anspruch 1, dadurch gekennzeichnet, daß sie (-)-(3S,4R, 5'R)-6-Cyano-3,4-dihydro-2,2-dimethyl-4-(2'-oxo-5'-methyl-1'-pyrrolidinyl)-2H-benzo[b]pyran-3-ol ist.

**12.** Verfahren zum Herstellen von Verbindungen I nach Anspruch 1, dadurch gekennzeichnet, daß man
a) Verbindungen der Formel II

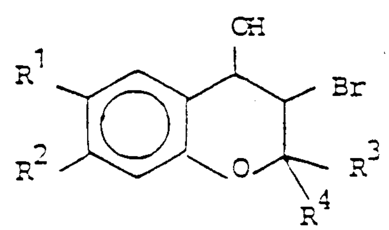

II,

in der $R^1$, $R^2$, $R^3$ und $R^4$ die obengenannten Bedeutungen haben,
umsetzt mit Lactamen der Formel III

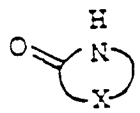

III,

oder

b) Verbindungen der Formel IV

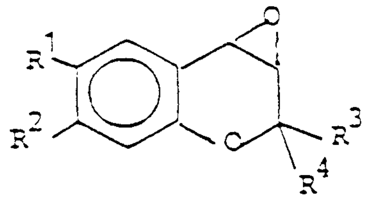

IV,

in der $R^1$, $R^2$, $R^3$ und $R^4$ die obengenannten Bedeutungen haben, umsetzt mit den Lactamen der Formel III,

oder

c) Verbindungen der Formel V

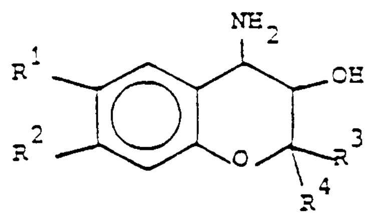

V,

in der $R^1$, $R^2$, $R^3$ und $R^4$ die obengenannten Bedeutungen haben, acyliert zu den Verbindungen VI

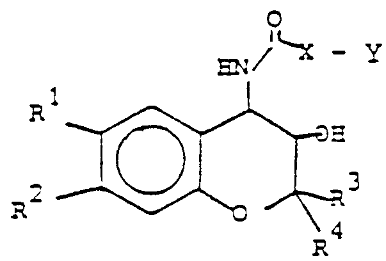

in denen $R^1$ bis $R^4$ und X wie oben definiert sind und Y die Bedeutung einer Fluchtgruppe hat, und diese cyclisiert zu den Verbindungen I,

oder

d) Verbindungen der Formel VII

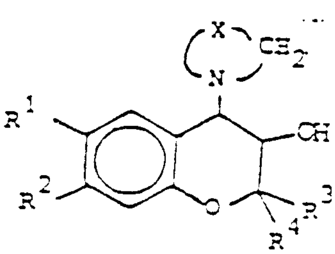

in der $R^1$ bis $R^4$ und X wie oben definiert sind, oxidiert zu den Verbindungen I.

**13.** Verwendung einer Verbindung der Formel I nach Anspruch 1 zur Herstellung eines Arzneimittels zur Behandlung des Bluthochdrucks.

**14.** Verwendung einer Verbindung der Formel I nach Ansprüchen 6, 8 oder 10 zur Herstellung eines Mittels zur Relaxation des Ureters.

**Patentansprüche für folgende Vertragsstaaten : ES, GR**

**1.** Verfahren zum Herstellen von N-Benzopyranyllactamen der Formel I

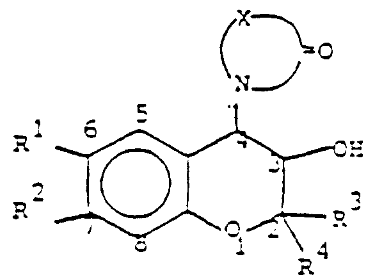

I,

in welcher

$R^1$ für CN, $NO_2$, $SO_n$-($C_1$-$C_6$)-Alkyl, $SO_n$-Ar, steht; wobei n = 1 oder 2, Ar für Phenyl steht, das unsubstituiert oder durch 1 bis 3 gleiche oder verschiedene Reste ($C_1$-$C_2$)-Alkyl, ($C_1$-$C_2$)Alkoxy, Halogen, Trifluormethyl, CN, $NO_2$, CO-($C_1$-$C_2$)-Alkyl, $SO_p$-($C_1$-$C_2$)-Alkyl, substituiert ist und p für 1 oder 2 steht,

$R^2$ für H, OH, ($C_1$-$C_2$)-Alkoxy, ($C_1$-$C_2$)-Alkyl, Halogen oder $NR^5R^6$ steht, wobei $R^5$ und $R^6$ gleich oder verschieden sind und für H, ($C_1$-$C_2$)Alkyl oder ($C_1$-$C_2$)-Alkylcarbonyl stehen,

$R^3$ und $R^4$ gleich oder verschieden sind und für Alkyl mit 1-4 C-Atomen stehen,

die oben genannten Bedeutungen von $R^1$ und $R^2$ auch vertauscht sein können,

X für eine Kette $(CH_2)_m$ steht, die durch mindestens 1 und höchstens 2m-1 ($C_1$-$C_2$)-Alkylgruppen substituiert ist, sowie durch ein Heteroatom Y in der Bedeutung von O, $NR^7$, S unterbrochen sein kann und $R^7$ H oder ($C_1$-$C_4$)-Alkyl bedeutet und m für 2, 3 oder 4 steht, wobei die Konfiguration an $C_3$ und $C_4$ stets entgegengesetzt ist,

dadurch gekennzeichnet, daß

a) Verbindungen der Formel II

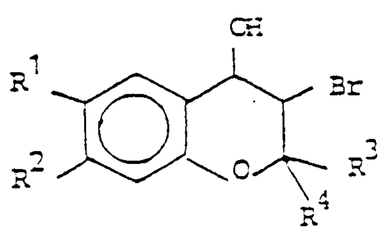

II,

in der $R^1$, $R^2$, $R^3$ und $R^4$ die obengenannten Bedeutungen haben,
umsetzt mit Lactamen der Formel III

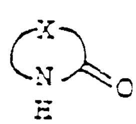

III,

oder

b) Verbindungen der Formel IV

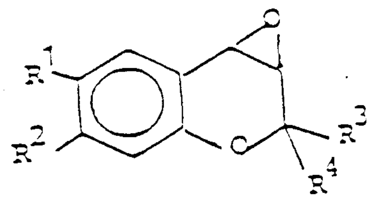

IV,

in der $R^1$, $R^2$, $R^3$ und $R^4$ die obengenannten Bedeutungen haben, umsetzt mit den Lactamen der Formel III,

oder

c) Verbindungen der Formel V

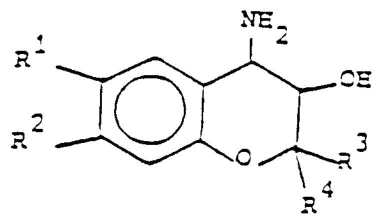

V,

in der $R^1$, $R^2$, $R^3$ und $R^4$ die obengenannten Bedeutungen haben, acyliert zu den Verbindungen VI

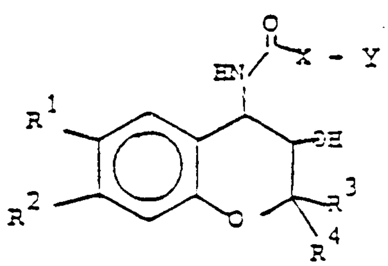

in denen $R^1$ bis $R^4$ und X wie oben definiert sind und Y die Bedeutung einer Fluchtgruppe hat, und diese cyclisiert zu den Verbindungen I,

oder

d) Verbindungen der Formel VII

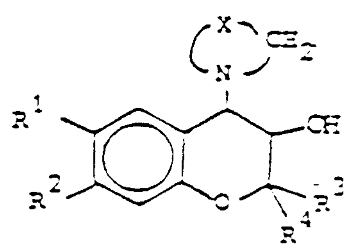

in der $R^1$ bis $R^4$ und X wie oben definiert sind, oxidiert zu den Verbindungen I.

2. Verfahren nach Anspruch 1, dadurch gekennzeichnet, daß mindestens einer der Substituenten oder Indices folgende Bedeutung hat:
$R^1$, $R^2$, $R^3$, $R^4$ wie in Anspruch 1 definiert,

X eine Kette $(CH_2)_m$, die durch eine $(C_1\text{-}C_2)$-Alkylgruppe substituiert ist, sowie durch ein Heteroatom Y, das für O, S oder $NR^7$ steht mit $R^7$ in der Bedeutung von H oder $(C_2\text{-}C_4)$-Alkyl, unterbrochen sein kann und wobei m für 2, 3 oder 4 steht.

**3.** Verfahren I nach Anspruch 1, dadurch gekennzeichnet, daß mindestens einer der Substituenten oder Indices folgende Bedeutung hat:
$R^1$ bis $R^4$ wie in Anspruch 1 definiert,
X eine Kette $(CH_2)_m$, die durch eine $(C_1\text{-}C_2)$-Alkylgruppe substituiert ist wobei m für 3 oder 4 steht.

**4.** Verfahren I nach Anspruch 1, dadurch gekennzeichnet, daß mindestens einer der Substituenten oder Indices folgende Bedeutung hat:
$R^1$ bis $R^4$ wie in Anspruch 1 definiert,
X eine Kette $(CH_2)_m$, wobei m für 3 oder 4 steht, die durch eine $(C_1\text{-}C_2)$-Alkylgruppe an dem C-Atom substituiert ist, das dem N-Atom des Lactamringes benachbart ist.

**5.** Verfahren nach Anspruch 1, dadurch gekennzeichnet, daß mindestens einer der Substituenten oder Indices folgende Bedeutung hat:
$R^1$-$R^4$ wie in Anspruch 1 definiert,
X eine Kette $(CH_2)_m$ mit m = 3 oder 4, die durch eine $(C_1\text{-}C_2)$-Alkylgruppe an dem C-Atom substituiert ist, das dem N-Atom des Lactamringes benachbart ist, wobei die Konfiguration dieses C-Atoms dieselbe ist wie die des C-4 Atom des Chromansystems.

**6.** Verfahren nach Anspruch 1, dadurch gekennzeichnet, daß mindestens einer der Substituenten oder Indices folgende Bedeutung hat:
$R^1$-$R^4$ wie in Anspruch 1 definiert,
X eine Kette $(CH_2)_m$ mit m = 3 oder 4, die durch eine $(C_1\text{-}C_2)$-Alkylgruppe an dem C-Atom substituiert ist, das dem N-Atom des Lactamringes benachbart ist, wobei die Konfiguration dieses C-Atoms entgegengesetzt ist zu der des C-4 Atom des Chromansystems.

**7.** Verfahren nach Anspruch 1, dadurch gekennzeichnet, daß mindestens einer der Substituenten oder Indices folgende Bedeutung hat:

| | |
|---|---|
| $R^1$ | CN, $SO_2CH_3$ |
| $R^2$ | H, |
| $R^3$ und $R^4$ | (gleich oder verschieden) $(C_1\text{-}C_2)$-Alkyl, |
| X | eine Kette $(CH_2)_m$ mit m = 3 oder 4, die durch eine $(C_1\text{-}C_2)$-Alkylgruppe an dem C-Atom substituiert ist, das dem N-Atom des Lactamringes benachbart ist, wobei die Konfiguration dieses C-Atoms dieselbe ist wie das des C-4 Atoms im Chromansystem. |

**8.** Verfahren nach Anspruch 1, dadurch gekennzeichnet, daß mindestens einer der Substituenten oder Indices folgende Bedeutung hat:

| | |
|---|---|
| $R^1$ | CN, $SO_2CH_3$ |
| $R^2$ | H, |
| $R^3$ und $R^4$ | (gleich oder verschieden) $(C_1\text{-}C_2)$-Alkyl, |
| X | eine Kette $(CH_2)_m$ mit m = 3 oder 4, die durch eine $(C_1\text{-}C_2)$-Alkylgruppe an dem C-Atom substituiert ist, das dem N-Atom des Lactamringes benachbart ist, wobei die Konfiguration dieses C-Atoms entgegengesetzt ist zu der des C-4 Atoms im Chromansystem. |

**9.** Verfahren nach Anspruch 1, dadurch gekennzeichnet, daß mindestens einer der Substituenten oder Indices folgende Bedeutung hat:

| | |
|---|---|
| $R^1$ | $SO_2Ar$ mit Ar gleich Phenyl, das unsubstituiert oder wie in Anspruch 1 definiert substituiert ist, |
| $R^2$ | H, $CH_3$, |
| $R^3$ und $R^4$ | (gleich oder verschieden) $(C_1\text{-}C_2)$-Alkyl, |
| X | $(CH_2)_m$ mit m = 3 oder 4, substituiert durch eine $(C_1\text{-}C_2)$-Alkylgruppe an dem C-Atom, das dem N-Atom des Lactamringes benachtbart ist, wobei die Konfiguration dieses C-Atoms dieselbe ist wie die des C-4-Atoms im Chromansystem. |

10. Verwendung einer Verbindung I nach Anspruch 1 zur Herstellung eines Medikaments zur Behandlung des Bluthochdrucks.

11. Verwendung einer Verbindung I nach Anspruch 1 zur Herstellung eines Medikaments zum Relaxieren des Ureters.

**Claims**

**Claims for the following Contracting States : AT, BE, CH, DE, FR, GB, IT, LI, LU, NL, SE**

1. An N-benzopyranyllactam of the formula I

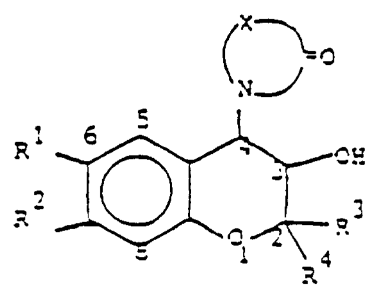

I,

in which

$R^1$ represents CN, $NO_2$, $SO_n$-($C_1$-$C_6$)-alkyl or $SO_n$-Ar; where n = 1 or 2, Ar represents phenyl which is unsubstituted or substituted by 1 to 3 identical or different ($C_1$-$C_2$)-alkyl, ($C_1$-$C_2$)-alkoxy, halogen, trifluoromethyl, CN, $NO_2$, CO-($C_1$-$C_2$)-alkyl or $SO_p$-($C_1$-$C_2$)-alkyl radicals, and p represents 1 or 2,

$R^2$ represents H, OH, ($C_1$-$C_2$)-alkoxy, ($C_1$-$C_2$)-alkyl, halogen or $NR^5R^6$, where $R^5$ and $R^6$ are identical or different and represent H, ($C_1$-$C_2$)-alkyl or ($C_1$-$C_2$)-alkylcarbonyl,

$R^3$ and $R^4$ are identical or different and represent alkyl having 1-4 carbon atoms,

the abovementioned meanings of $R^1$ and $R^2$ may also be exchanged, and

X represents a $(CH_2)_m$ chain which is substituted by at least 1 and at most 2m-1 ($C_1$-$C_2$)-alkyl groups and may be interrupted by a heteroatom Y denoting O, $NR^7$ or S, and $R^7$ represents H or ($C_1$-$C_4$)-alkyl, and m represents 2, 3 or 4, where the configurations at $C_3$ and $C_4$ are always opposed.

2. A compound I as claimed in claim 1, wherein at least one of the substituents or indices has the following meaning:
$R^1$, $R^2$, $R^3$ and $R^4$ are as defined in claim 1,
X is a $(CH_2)_m$ chain which is substituted by a ($C_1$-$C_2$)alkyl group and may be interrupted by a heteroatom Y which represents O, S or $NR^7$ where $R^7$ denotes H or ($C_2$-$C_4$)-alkyl, and where m represents 2, 3 or 4.

3. A compound I as claimed in claim 1, wherein at least one of the substituents or indices has the following meaning:
$R^1$ to $R^4$ are as defined in claim 1,
X is a $(CH_2)_m$ chain which is substituted by a ($C_1$-$C_2$)-alkyl group and where m represents 3 or 4.

4. A compound I as claimed in claim 1, wherein at least one of the substituents or indices has the following meaning:
$R^1$ to $R^4$ are as defined in claim 1,
X is a $(CH_2)_m$ chain where m represents 3 or 4 and which is substituted on the carbon atom neighboring the nitrogen atom of the lactam ring by a ($C_1$-$C_2$)-alkyl group.

5. A compound I as claimed in claim 1, wherein at least one of the substituents or indices has the following meaning:
$R^1$-$R^4$ are as defined in claim 1,
X is a $(CH_2)_m$ chain where m = 3 or 4 and which is substituted on the carbon atom neighboring the

nitrogen atom of the lactam ring by a ($C_1$-$C_2$)-alkyl group, the configuration of this carbon atom being the same as that of the C-4 atom of the chroman system.

**6.** A compound I as claimed in claim 1, wherein at least one of the substituents or indices has the following meaning:
$R^1$-$R^4$ are as defined in claim 1,
X is a $(CH_2)_m$ chain where m = 3 or 4 and which is substituted on the carbon atom neighboring the nitrogen atom of the lactam ring by a ($C_1$-$C_2$)-alkyl group, the configuration of this carbon atom being opposite to that of the C-4 atom of the chroman system.

**7.** A compound I as claimed in claim 1, wherein at least one of the substituents or indices has the following meaning:

| | |
|---|---|
| $R^1$ | denotes CN or $SO_2CH_3$, |
| $R^2$ | denotes H, |
| $R^3$ and $R^4$ | are identical or different and denote ($C_2$-$C_2$)-alkyl, and |
| X | denotes a $(CH_2)_m$ chain where m = 3 or 4 and which is substituted on the carbon atom neighboring the nitrogen atom of the lactam ring by a ($C_1$-$C_2$)-alkyl group, the configuration of this carbon atom being the same as that of the C-4 atom in the chroman system. |

**8.** A compound I as claimed in claim 1, wherein at least one of the substituents or indices has the following meaning:

| | |
|---|---|
| $R^1$ | denotes CN or $SO_2CH_3$, |
| $R^2$ | denotes H, |
| $R^3$ and $R^4$ | are identical or different and denote ($C_1$-$C_2$)-alkyl, and |
| X | denotes a $(CH_2)_m$ chain where m = 3 or 4 and which is substituted on the carbon atom neighboring the nitrogen atom of the lactam ring by a ($C_1$-$C_2$)-alkyl group, the configuration of this carbon atom being opposite to that of the C-4 atom in the chroman system. |

**9.** A compound I as claimed in claim 1, wherein at least one of the substituents or indices has the following meaning:

| | |
|---|---|
| $R^1$ | denotes $SO_2Ar$ where Ar equals phenyl which is unsubstituted or substituted as defined in claim 1, |
| $R^2$ | denotes H or $CH_3$, |
| $R^3$ and $R^4$ | are identical or different and denote ($C_1$-$C_2$)-alkyl, and |
| X | denotes $(CH_2)_m$ where m = 3 or 4, substituted on the carbon atom neighboring the nitrogen atom of the lactam ring by a ($C_1$-$C_2$)-alkyl group, the configuration of this carbon atom being the same as that of the C-4 atom in the chroman system. |

**10.** A compound as claimed in claim 1, which is (-)-(3R,-4S, 5'R)-6-cyano-3,4-dihydro-2,2-dimethyl-4-(2'-oxo-5'-methyl-1-pyrrolidinyl)-2H-benzo[b]pyran-3-ol.

**11.** A compound as claimed in claim 1, which is (-)-(3S,-4R,5'R)-6-cyano-3,4-dihydro-2,2-dimethyl-4-(2'-oxo-5'-methyl-1'-pyrrolidinyl)-2H-benzo[b]pyran-3-ol.

**12.** A process for the preparation of a compound I as claimed in claim 1, which comprises
a) reacting a compound of the formula II

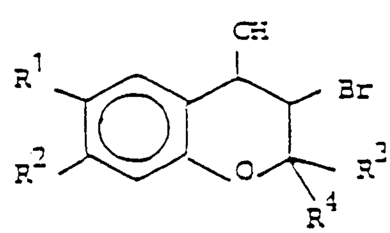

II,

in which $R^1$, $R^2$, $R^3$ and $R^4$ have the abovementioned meanings, with a lactam of the formula III

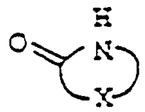

or

b) reacting a compound of the formula IV

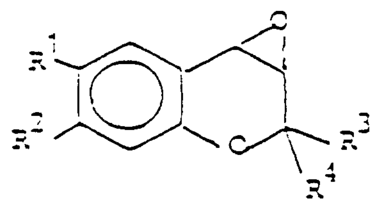

IV,

in which $R^1$, $R^2$, $R^3$ and $R^4$ have the abovementioned meanings, with a lactam of the formula III,

or

c) acylating a compound of the formula V

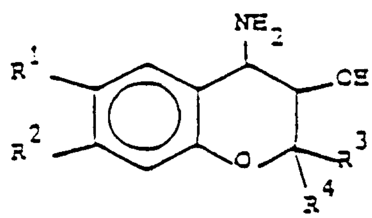

V,

in which $R^1$, $R^2$, $R^3$ and $R^4$ have the abovementioned meanings, to give a compound VI

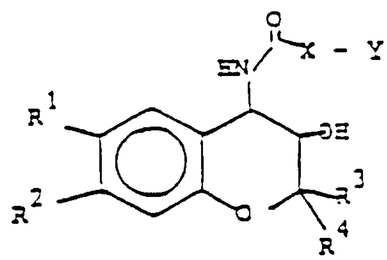

in which $R^1$ to $R^4$ and X are as defined above and Y denotes a leaving group, and cyclizing the latter to give a compound I,

or

d) oxidizing a compound of the formula VII

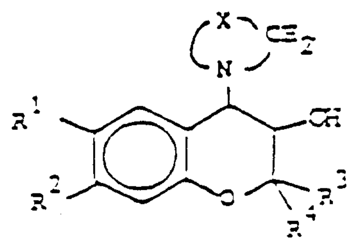

in which $R^1$ to $R^4$ and X are as defined above, to give a compound I.

**13.** The use of a compound of the formula I as claimed in claim 1 for the production of a medicament for treating high blood pressure.

**14.** The use of a compound of the formula I as claimed in any of claims 6, 8 and 10, for the production of an agent for relaxation of the ureter.

**Claims for the following Contracting States : ES, GR**

**1.** A process for the preparation of N-benzopyranyllactams of the formula I

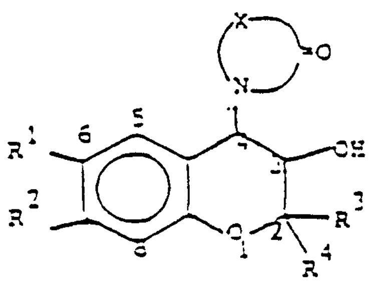

I,

in which

$R^1$ represents CN, $NO_2$, $SO_n$-($C_1$-$C_6$)-alkyl or $SO_n$-Ar, where n = 1 or 2, Ar represents phenyl which is unsubstituted or substituted by 1 to 3 identical or different ($C_1$-$C_2$)-alkyl, ($C_1$-$C_2$)alkoxy, halogen, trifluoromethyl, CN, $NO_2$, CO-($C_1$-$C_2$)-alkyl or $SO_p$-($C_1$-$C_2$)-alkyl radicals, and p represents 1 or 2,

$R^2$ represents H, OH, ($C_1$-$C_2$)-alkoxy, ($C_1$-$C_2$)-alkyl, halogen or $NR^5R^6$, where $R^5$ and $R^6$ are identical or different and represent H, ($C_1$-$C_2$)-alkyl or ($C_1$-$C_2$)-alkylcarbonyl,

$R^3$ and $R^4$ are identical or different and represent alkyl having 1-4 carbon atoms,

the abovementioned meanings of $R^1$ and $R^2$ may also be exchanged, and

X represents a $(CH_2)_m$ chain which is substituted by at least 1 and at most 2m-1 ($C_1$-$C_2$)-alkyl groups and may be interrupted by a heteroatom Y denoting O, $NR^7$ or S, and $R^7$ represents H or ($C_1$-$C_4$)-alkyl, and m represents 2, 3 or 4, where the configurations at $C_3$ and $C_4$ are always opposite to each other, which comprises

a) reacting a compound of the formula II

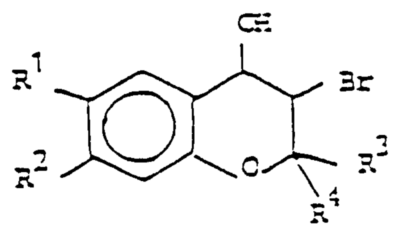

II,

in which $R^1$, $R^2$, $R^3$ and $R^4$ have the abovementioned meanings, with a lactam of the formula III

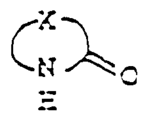

III,

or

b) reacting a compound of the formula IV

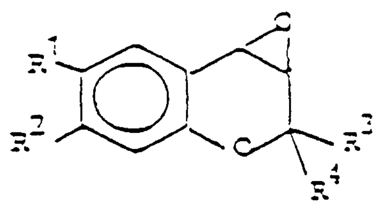

IV,

in which $R^1$, $R^2$, $R^3$ and $R^4$ have the abovementioned meanings, with a lactam of the formula III,

or

c) acylating a compound of the formula V

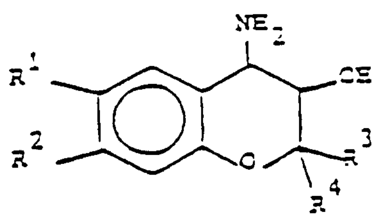

V,

in which $R^1$, $R^2$, $R^3$ and $R^4$ have the abovementioned meanings, to give a compound VI

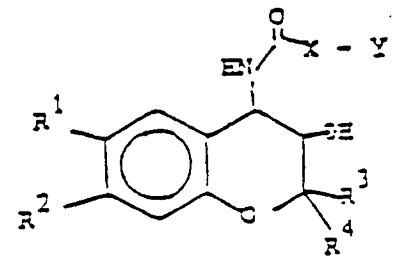

in which $R^1$ to $R^4$ and X are as defined above and Y denotes a leaving group, and cyclizing the latter to give a compound I,

or

d) oxidizing a compound of the formula VII

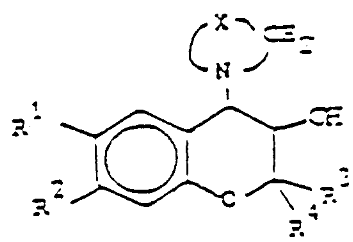

in which $R^1$ to $R^4$ and X are as defined above, to give a compound I.

**2.** A process as claimed in claim 1, wherein at least one of the substituents or indices has the following meaning:
$R^1$, $R^2$, $R^3$ and $R^4$ are as defined in claim 1,
X is a $(CH_2)_m$ chain which is substituted by a $(C_1\text{-}C_2)$-alkyl group and may be interrupted by a heteroatom Y which represents O, S or $NR^7$ where $R^7$ denotes H or $(C_2\text{-}C_4)$-alkyl, and where m represents 2, 3 or 4.

**3.** A process I as claimed in claim 1, wherein at least one of the substituents or indices has the following meaning:
$R^1$ to $R^4$ are as defined in claim 1,
X is a $(CH_2)_m$ chain which is substituted by a $(C_1\text{-}C_2)$-alkyl group and where m represents 3 or 4.

**4.** A process I as claimed in claim 1, wherein at least one of the substituents or indices has the following meaning:
$R^1$ to $R^4$ are as defined in claim 1,
X is a $(CH_2)_m$ chain where m represents 3 or 4 and which is substituted on the carbon atom neighboring the nitrogen atom of the lactam ring by a $(C_1\text{-}C_2)$-alkyl group.

**5.** A process as claimed in claim 1, wherein at least one of the substituents or indices has the following meaning:
$R^1$-$R^4$ are as defined in claim 1,
X is a $(CH_2)_m$ chain where m = 3 or 4 and which is substituted on the carbon atom neighboring the nitrogen atom of the lactam ring by a $(C_1\text{-}C_2)$-alkyl group, the configuration of this carbon atom being the same as that of the C-4 atom of the chroman system.

**6.** A process as claimed in claim 1, wherein at least one of the substituents or indices has the following meaning:
$R^1$-$R^4$ are as defined in claim 1,
X is a $(CH_2)_m$ chain where m = 3 or 4 and which is substituted on the carbon atom neighboring the

nitrogen atom of the lactam ring by a ($C_1$-$C_2$)-alkyl group, the configuration of this carbon atom being opposite to that of the C-4 atom of the chroman system.

**7.** A process as claimed in claim 1, wherein at least one of the substituents or indices has the following meaning:
- $R^1$ denotes CN or $SO_2CH_3$,
- $R^2$ denotes H,
- $R^3$ and $R^4$ are identical or different and denote ($C_1$-$C_2$)-alkyl, and
- X denotes a $(CH_2)_m$ chain where m = 3 or 4 and which is substituted on the carbon atom neighboring the nitrogen atom of the lactam ring by a ($C_1$-$C_2$)-alkyl group, the configuration of this carbon atom being the same as that of the C-4 atom in the chroman system.

**8.** A process as claimed in claim 1, wherein at least one of the substituents or indices has the following meaning:
- $R^1$ denotes CN or $SO_2CH_3$,
- $R^2$ denotes H,
- $R^3$ and $R^4$ are identical or different and denote ($C_1$-$C_2$)-alkyl, and
- X denotes a $(CH_2)_m$ chain where m = 3 or 4 and which is substituted on the carbon atom neighboring the nitrogen atom of the lactam ring by a ($C_1$-$C_2$)-alkyl group, the configuration of this carbon atom being opposite to that of the C-4 atom in the chroman system.

**9.** A process as claimed in claim 1, wherein at least one of the substituents or indices has the following meaning:
- $R^1$ denotes $SO_2Ar$ where Ar equals phenyl which is unsubstituted or substituted as defined in claim 1,
- $R^2$ denotes H or $CH_3$,
- $R^3$ and $R^4$ are identical or different and denote ($C_1$-$C_2$)-alkyl, and
- X denotes $(CH_2)_m$ where m = 3 or 4, substituted on the carbon atom neighboring the nitrogen atom of the lactam ring by a ($C_1$-$C_2$)-alkyl group, the configuration of this carbon atom being the same as that of the C-4 atom in the chroman system.

**10.** The use of a compound I as claimed in claim 1 for the production of a medicament for treating high blood pressure.

**11.** The use of a compound I as claimed in claim 1 for the production of a medicament for relaxation of the ureter.

## Revendications
## Revendications pour les Etats contractants suivants : AT, BE, CH, DE, FR, GB, IT, LI, LU, NL, SE

**1.** N-benzopyrannyl-lactames de formule I

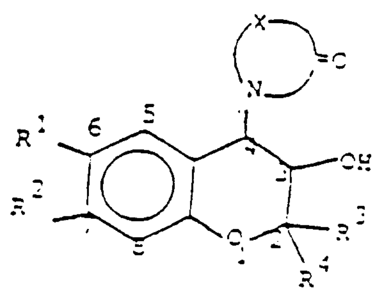

I

dans laquelle
- $R^1$ représente CN, $NO_2$, un groupe $SO_n$-alkyle($C_1$-$C_6$), $SO_n$-Ar, n étant 1 ou 2, Ar représentant un groupe phényle qui est non substitué ou substitué par 1 à 3 substituants, identiques ou différents, alkyle en $C_1$-$C_2$, alcoxy en $C_1$-$C_2$, halogène, trifluorométhyle, CN, $NO_2$, CO-alkyle($C_1$-$C_2$), $SO_p$-alkyle($C_1$-$C_2$) et p représentant 1 ou 2,

$R^2$ représente H, OH, un groupe alcoxy en $C_1$-$C_2$, alkyle en $C_1$-$C_2$, un atome d'halogène ou $NR^5R^6$, $R^5$ et $R^6$ étant identiques ou différents et représentant H, un groupe alkyle en $C_1$-$C_2$ ou alkyl($C_1$-$C_2$)-carbonyle,

$R^3$ et $R^4$ sont identiques ou différents et représentent un groupe alkyle ayant de 1 à 4 atomes de carbone,

les significations de $R^1$ et $R^2$ données plus haut peuvent également être interverties,

X représente une chaîne $(CH_2)_m$ qui est substituée par au moins 1 et au plus 2m-1 groupes alkyle en $C_1$-$C_2$, et peut être interrompue par un hétéroatome Y représentant O, $NR^7$, S, et $R^7$ représentant H ou un groupe alkyle en $C_1$-$C_4$, et m représentant 2, 3 ou 4,

les configurations en $C_3$ et $C_4$ étant toujours inverses.

**2.** Composé I selon la revendication 1, caractérisé en ce qu'au moins l'un des substituants ou indices a la signification suivante:
$R^1$, $R^2$, $R^3$, $R^4$ sont tels que définis dans la revendication 1,
X représente une chaîne $(CH_2)_m$ qui est substituée par un groupe alkyle en $C_1$-$C_2$ et peut être interrompue par un hétéroatome Y qui représente O, S ou $NR^7$, $R^7$ représentant H ou un groupe alkyle en $C_2$-$C_4$, et m représentant 2, 3 ou 4.

**3.** Composé I selon la revendication 1, caractérisé en ce qu'au moins l'un des substituants ou indices a la signification suivante:
$R^1$ à $R^4$ sont tels que définis dans la revendication 1,
X représente une chaîne $(CH_2)_m$ qui est substituée par un groupe alkyle en $C_1$-$C_2$ et dans laquelle m représente 3 ou 4.

**4.** Composé I selon la revendication 1, caractérisé en ce qu'au moins l'un des substituants ou indices a la signification suivante:
$R^1$ à $R^4$ sont tels que définis dans la revendication 1,
X représente une chaîne $(CH_2)_m$, m représentant 3 ou 4, qui est substituée par un groupe alkyle en $C_1$-$C_2$ sur l'atome de carbone qui est contigu à l'atome d'azote du cycle lactame.

**5.** Composé I selon la revendication 1, caractérisé en ce qu'au moins l'un des substituants ou indices a la signification suivante:
$R^1$-$R^4$ sont tels que définis dans la revendication 1,
X représente une chaîne $(CH_2)$m, avec m = 3 ou 4, qui est substituée par un groupe alkyle en $C_1$-$C_2$ sur l'atome de carbone qui est contigu à l'atome d'azote du cycle lactame, la configuration de cet atome de carbone étant la même que celle de l'atome de carbone 4 du système chromane.

**6.** Composé I selon la revendication 1, caractérisé en ce qu'au moins l'un des substituants ou indices a la signification suivante:
$R^1$-$R^4$ sont tels que définis dans la revendication 1,
X représente une chaîne $(CH_2)_m$, avec m = 3 ou 4, qui est substituée par un groupe alkyle en $C_1$-$C_2$ sur l'atome de carbone qui est contigu à l'atome d'azote du cycle lactame, la configuration de cet atome de carbone étant inverse de celle de l'atome de carbone 4 du système chromane.

**7.** Composé I selon la revendication 1, caractérisé en ce qu'au moins l'un des substituants ou indices a la signification suivante:

$R^1$ représente CN, $SO_2$-$CH_3$,

$R^2$ représente H,

$R^3$ et $R^4$ (identiques ou différents) représentent un groupe alkyle en $C_1$-$C_2$,

X représente une chaîne $(CH_2)_m$ avec m = 3 ou 4, qui est substituée par un groupe alkyle en $C_1$-$C_2$ sur l'atome de carbone qui est contigu à l'atome d'azote du cycle lactame, la configuration de cet atome de carbone étant la même que celle de l'atome de carbone 4 dans le système chromane.

8. Composé I selon la revendication 1, caractérisé en ce qu'au moins l'un des substituants ou indices a la signification suivante:

| | |
|---|---|
| $R^1$ | représente CN, $SO_2$-$CH_3$, |
| $R^2$ | représente H, |
| $R^3$ et $R^4$ | (identiques ou différents) représentent un groupe alkyle en $C_1$-$C_2$, |
| X | représente une chaîne $(CH_2)_m$ avec m = 3 ou 4, qui est substituée par un groupe alkyle en $C_1$-$C_2$ sur l'atome de carbone qui est contigu à l'atome d'azote du cycle lactame, la configuration de cet atome de carbone étant inverse de celle de l'atome de carbone 4 dans le système chromane. |

9. Composé I selon la revendication 1, caractérisé en ce qu'au moins l'un des substituants ou indices a la signification suivante:

| | |
|---|---|
| $R^1$ | représente $SO_2$Ar, Ar étant un groupe phényle qui est non substitué ou substitué comme défini dans la revendication 1, |
| $R^2$ | représente H, $CH_3$, |
| $R^3$ et $R^4$ | (identiques ou différents) représentent un groupe alkyle en $C_1$-$C_2$, |
| X | représente une chaîne $(CH_2)_m$ avec m = 3 ou 4, qui est substituee par un groupe alkyle en $C_1$-$C_2$ sur l'atome de carbone qui est contigu à l'atome d'azote du cycle lactame, la configuration de cet atome de carbone étant la même que celle de l'atome de carbone 4 dans le système chromane. |

10. Composé selon la revendication 1, caractérisé en ce qu'il est le (-)-(3R,4S,5'R)-6-cyano-3,4-dihydro-2,2-diméthyl-4-(2'-oxo-5'-méthyl-1-pyrrolidinyl)-2H-benzo[b]-pyranne-3-ol.

11. Composé selon la revendication 1, caractérisé en ce qu'il est le (-)-(3R,4R,5'R)-6-cyano-3,4-dihydro-2,2-diméthyl-4-(2'-oxo-5'-méthyl-1'-pyrrolidinyl)-2H-benzo[b]-pyranne-3-ol.

12. Procédé pour la préparation de composés I selon la revendication 1, caractérisé en ce que

a) on fait réagir des composés de formule II

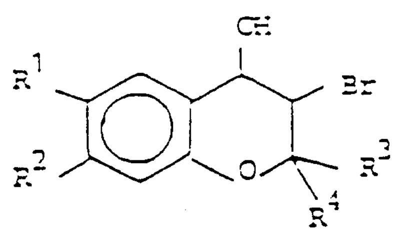

II

dans laquelle $R^1$, $R^2$, $R^3$ et $R^4$ ont les significations données plus haut, avec des lactames de formule III

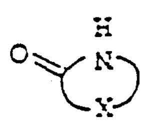

III

ou

b) on fait réagir des composés de formule IV

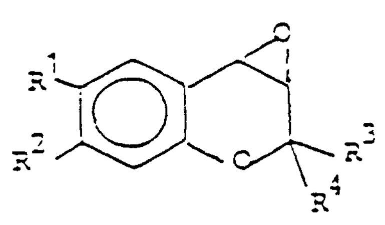

IV

dans laquelle $R^1$, $R^2$, $R^3$ et $R^4$ ont les significations don nées plus haut,
avec les lactames de formule III,
ou
c) on soumet des composés de formule V

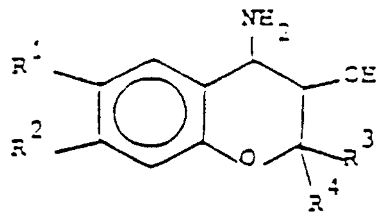

V

dans laquelle $R^1$, $R^2$, $R^3$ et $R^4$ ont les significations données plus haut,
à une acylation conduisant aux composés de formule VI

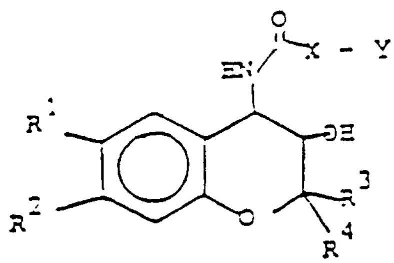

dans laquelle $R^1$ à $R^4$ et X sont tels que définis plus haut et Y a la signification d'un groupe séparable,
et on les soumet à une cyclisation pour aboutir aux composés I,
d) on oxyde des composés de formule VII

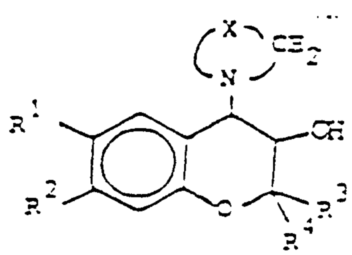

VII

dans laquelle $R^1$ à $R^4$ et X sont tels que définis plus haut, pour aboutir aux composés I.

**13.** Utilisation d'un composé de formule I selon la revendication 1, pour la fabrication d'un médicament pour le traitement de l'hypertension.

**14.** Utilisation d'un composé de formule I selon les revendications 6, 8 ou 10, pour la fabrication d'un agent pour le relâchement de l'uretère.

**Revendications pour les Etats contractants suivants : ES, GR**

**1.** Procédé pour la préparation de N-benzopyrannyllactames de formule I

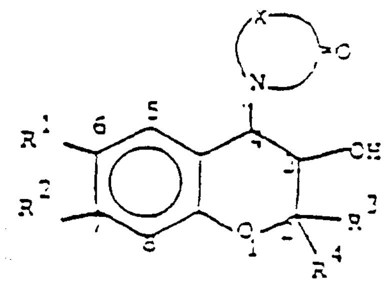

I

dans laquelle

- $R^1$ représente CN, $NO_2$, un groupe $SO_n$-alkyle($C_1$-$C_6$), $SO_n$-Ar, n étant 1 ou 2, Ar représentant un groupe phényle qui est non substitué ou substitué par 1 à 3 substituants, identiques ou différents, alkyle en $C_1$-$C_2$, alcoxy en $C_1$-$C_2$, halogène, trifluorométhyle, CN, $NO_2$, CO-alkyle($C_1$-$C_2$), $SO_p$-alkyle($C_1$-$C_2$) et p représentant 1 ou 2,
- $R^2$ représente H, OH, un groupe alcoxy en $C_1$-$C_2$, alkyle en $C_1$-$C_2$, un atome d'halogène ou $NR^5R^6$, $R^5$ et $R^6$ étant identiques ou différents et représentant H, un groupe alkyle en $C_1$-$C_2$ ou alkyl($C_1$-$C_2$)-carbonyle,
- $R^3$ et $R^4$ sont identiques ou différents et représentent un groupe alkyle ayant de 1 à 4 atomes de carbone,

les significations de $R^1$ et $R^2$ données plus haut peuvent également être interverties,

- X représente une chaîne $(CH_2)_m$ qui est substituée par au moins 1 et au plus 2m-1 groupes alkyle en $C_1$-$C_2$, et peut être interrompue par un hétéroatome Y représentant O, $NR^7$, S, et $R^7$ représentant H ou un groupe alkyle en $C_1$-$C_4$, et m représentant 2, 3 ou 4,

les configurations en $C_3$ et $C_4$ étant toujours inverses,
caractérisé en ce que

a) on fait réagir des composés de formule II

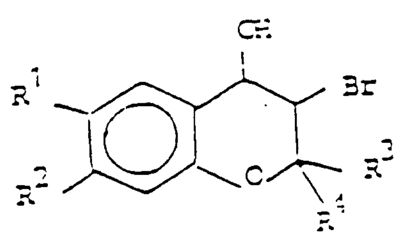

II

dans laquelle $R^1$, $R^2$, $R^3$ et $R^4$ ont les significations données plus haut,
avec des lactames de formule III

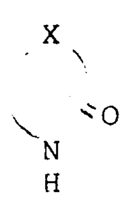

III

ou

b) on fait réagir des composés de formule IV

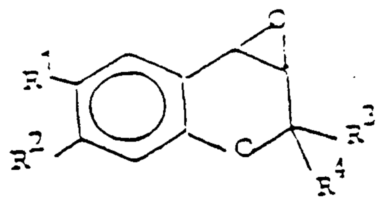

IV

dans laquelle $R^1$, $R^2$, $R^3$ et $R^4$ ont les significations données plus haut,
avec les lactames de formule III,
ou
c) on soumet des composés de formule V

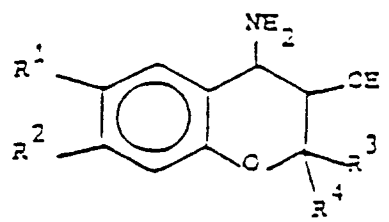

V

dans laquelle $R^1$, $R^2$, $R^3$ et $R^4$ ont les significations données plus haut,
à une acylation conduisant aux composés de formule VI

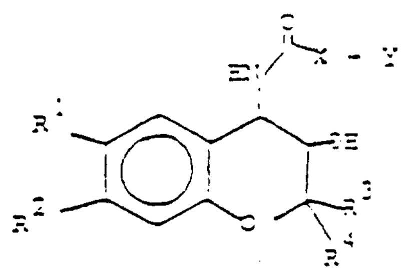

dans laquelle $R^1$ à $R^4$ et X sont tels que définis plus haut et Y a la signification d'un groupe séparable,
et on les soumet à une cyclisation pour aboutir aux composés I,
d) on oxyde des composés de formule VII

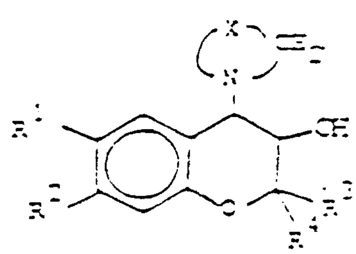

dans laquelle $R^1$ à $R^4$ et X sont tels que définis plus haut, pour aboutir aux composés I.

**2.** Procédé selon la revendication 1, caractérisé en ce qu'au moins l'un des substituants ou indices a la signification suivante:
$R^1$, $R^2$, $R^3$, $R^4$ sont tels que définis dans la revendication 1,
X représente une chaîne $(CH_2)_m$ qui est substituée par un groupe alkyle en $C_1$-$C_2$ et peut être interrompue par un hétéroatome Y qui représente O, S ou $NR^7$, $R^7$ représentant H ou un groupe alkyle en $C_2$-$C_4$, et m représentant 2, 3 ou 4.

**3.** Procédé selon la revendication 1, caractérisé en ce qu'au moins l'un des substituants ou indices a la signification suivante:
$R^1$ à $R^4$ sont tels que définis dans la revendication 1,
X représente une chaîne $(CH_2)_m$ qui est substituée par un groupe alkyle en $C_1$-$C_2$ et dans laquelle m représente 3 ou 4.

**4.** Procédé selon la revendication 1, caractérisé en ce qu'au moins l'un des substituants ou indices a la signification suivante:
$R^1$ à $R^4$ sont tels que définis dans la revendication 1,
X représente une chaîne $(CH_2)_m$, m représentant 3 ou 4, qui est substituée par un groupe alkyle en $C_1$-$C_2$ sur l'atome de carbone qui est contigu à l'atome d'azote du cycle lactame.

**5.** Procédé selon la revendication 1, caractérisé en ce qu'au moins l'un des substituants ou indices a la signification suivante:
$R^1$-$R^4$ sont tels que définis dans la revendication 1,
X représente une chaîne $(CH_2)_m$, avec m = 3 ou 4, qui est substituée par un groupe alkyle en $C_1$-$C_2$ sur l'atome de carbone qui est contigu à l'atome d'azote du cycle lactame, la configuration de cet atome de carbone étant la même que celle de l'atome de carbone 4 du système chromane.

**6.** Procédé selon la revendication 1, caractérisé en ce qu'au moins l'un des substituants ou indices a la signification suivante:
$R^1$-$R^4$ sont tels que définis dans la revendication 1,
X représente une chaîne $(CH_2)_m$, avec m = 3 ou 4, qui est substituée par un groupe alkyle en $C_1$-$C_2$ sur l'atome de carbone qui est contigu à l'atome d'azote du cycle lactame, la configuration de cet atome de carbone étant inverse de celle de l'atome de carbone 4 du système chromane.

**7.** Procédé selon la revendication 1, caractérisé en ce qu'au moins l'un des substituants ou indices a la signification suivante:

| | |
|---|---|
| $R^1$ | représente CN, $SO_2$-$CH_3$, |
| $R^2$ | représente H, |
| $R^3$ et $R^4$ | (identiques ou différents) représentent un groupe alkyle en $C_1$-$C_2$, |
| X | représente une chaîne $(CH_2)_m$ avec m = 3 ou 4, qui est substituée par un groupe alkyle en $C_1$-$C_2$ sur l'atome de carbone qui est contigu à l'atome d'azote du cycle lactame, la configuration de cet atome de carbone étant la même que celle de l'atome de carbone 4 dans le système chromane. |

**8.** Procédé selon la revendication 1, caractérisé en ce qu'au moins l'un des substituants ou indices a la signification suivante:

| | |
|---|---|
| $R^1$ | représente CN, $SO_2$-$CH_3$, |
| $R^2$ | représente H, |
| $R^3$ et $R^4$ | (identiques ou différents) représentent un groupe alkyle en $C_1$-$C_2$, |
| X | représente une chaîne $(CH_2)_m$ avec m = 3 ou 4, qui est substituée par un groupe alkyle en $C_1$-$C_2$ sur l'atome de carbone qui est contigu à l'atome d'azote du cycle lactame, la configuration de cet atome de carbone étant inverse de celle de l'atome de carbone 4 dans le système chromane. |

**9.** Procédé selon la revendication 1, caractérisé en ce qu'au moins l'un des substituants ou indices a la signification suivante:

| | |
|---|---|
| $R^1$ | représente $SO_2Ar$, Ar étant un groupe phényle qui est non substitué ou substitué comme défini dans la revendication 1, |
| $R^2$ | représente H, $CH_3$, |

$R^3$ et $R^4$ (identiques ou différents) représentent un groupe alkyle en $C_1$-$C_2$,

X représente une chaîne $(CH_2)_m$ avec m = 3 ou 4, qui est substituée par un groupe alkyle en $C_1$-$C_2$ sur l'atome de carbone qui est contigu à l'atome d'azote du cycle lactame, la configuration de cet atome de carbone étant la même que celle de l'atome de carbone 4 dans le système chromane.

**10.** Utilisation d'un composé de formule I selon la revendication 1, pour la fabrication d'un médicament pour le traitement de l'hypertension.

**11.** Utilisation d'un composé de formule I selon la revendication 1, pour la fabrication d'un agent pour le relâchement de l'uretère.